# EUROPEAN PATENT SPECIFICATION

(11) **EP 4 378 382 B1**
(45) Date of publication and mention of the grant of the patent: **09.10.2024**
(21) Application number: 22210869.8
(22) Date of filing: 01.12.2022
(51) Int. Cl.: A61B 5/0205, A61B 5/053, A61B 5/282, A61B 5/349, A61B 5/00, A61B 7/00, G16H 50/00

(54) **MULTIMODAL MEASUREMENT DEVICE AND SYSTEM**
MULTIMODALE MESSVORRICHTUNG UND SYSTEM
DISPOSITIF ET SYSTÈME DE MESURE MULTIMODALE

(43) Date of publication of application: 05.06.2024
(73) Proprietor: Lynx Health Science GmbH, 57439 Attendorn (DE)
(72) Inventor: Klum, Michael, 13581 Berlin (DE)
(74) Representative: Gulde & Partner

(56) References cited:
- US-A1- 2008 234 594
- US-A1- 2018 325 407
- US-A1- 2020 352 510
- KLUM MICHAEL ET AL: "Wearable Multimodal Stethoscope Patch for Wireless Biosignal Acquisition and Long-Term Auscultation", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23), pages 5781 - 5785, XP033625125, DOI: 10.1109/EMBC.2019.8857210

## Description

### Field of the Invention

The invention relates to the field of monitoring the activity of a body part of a subject.

### Technological Background

Monitoring the activity of a body part is essential for assessing the well-being or the fitness of a subject, such as a human being, tested and for determining a possible medical procedure to be administered thereto. Such procedures could be beneficial and prescribed to address or assess a specific concern, during a fitness/well-being assessment, or following a medical procedure for assessing the results of that procedure or the recovery process therefrom.

Complex measurement equipment devices are used to achieve high-accuracy measurements and commonly result in higher confidence in the measured endpoints. Such complex measurement equipment devices require high operation workloads, specialized/trained operators, and significant resources and space to operate. Additionally, such complex measurement equipment devices are not practical for long-term monitoring of patients, for example, in a home setting.

On the other hand, there are wearable devices, such as smart watches, that measure certain bio-signal activities with greater convenience, alas, while compromising the quality of the measured signals and the modalities that are measured. In similar regards, publication US 2020/352510 A1 relates to a wearable patch for monitoring vital signs and hemodynamic parameters using a plurality of sensors including ECG, IPG, PPG, and PCG, publication US 2018/325407 A1 relates to a wearable device for acquiring signals by placing a plurality of sensors on the body of the user by utilizing a harness including a vertical elastic band and a horizontal elastic band for placing the sensor at desired locations on the user, publication US 2008/234594 A1 relates to a device for collecting multiple signals, particularly acoustic signals, ICG, and ECG, for identifying certain markers in cardiac activity, and publication "KLUM MICHAEL ET AL: "Wearable Multimodal Stethoscope Patch for Wireless Biosignal Acquisition and Long-Term Auscultation", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23), pages 5781-5785" relates to a wearable multimodal stethoscope patch for wireless bio-signal acquisition and long-term auscultation.

There is thus a need in the art for measurement devices that facilitate convenient operation while providing high-quality measurements and assessments of the fitness and/or condition of the monitored body part of a subject.

### Summary of the Invention

The invention is set out in the appended set of claims.

### Summary of the Disclosure

According to an aspect of the present disclosure, there is provided a device for measuring and/or monitoring biological signals of a body part of a subject, corresponding to the appended claims. The device includes a surface electrical potential differentials sensor, electro-gram, EG, configured to measure electrical activity of the body part and to provide at least one EG signal indicative of the electrical activity of the body part, a surface vibrations sensor, phono-gram, PG, configured to measure an acoustic activity of the body part and to provide a PG signal indicative of the acoustic activity of the body part, and an impedance sensor, impedance-gram, IG, configured to measure an electrical impedance of the body part and to provide an IG signal indicative of the electrical impedance of the body part.

Advantageously, the device including an EG sensor, a PG sensor, and an IG sensor enables analyzing an EG signal, a PG signal, and an IG signal conjointly for deriving at least one datum based on all of the EG signal, the PG signal, and the IG signal.

As used herein, the term "subject" refers to a body on which the measurements are applied, such as a human being or an animal, such as a mammal, reptile, or bird. The terms "human", "subject", "user" (to which the device is applied, such as a patient or a subject), mammal", "body", "animal", "reptiles", and "birds" are interchangeably used.

As used herein, the wording "an acoustic activity of the body part" refers to a mechanical activity, vibrational activity, gas flow activity, fluid flow activity, or the like or any combination thereof. Advantageously, the device including an EG sensor, a PG sensor, and an IG sensor enables analyzing an EG signal, a PG signal, and an IG signal conjointly for deriving at least one predefined datum based on all of the EG signal, the PG signal, and the IG signal.

As used herein, the term "datum" refers to information intelligible for a human and/or a machine, the datum being indicative, directly or indirectly, of an existing condition or status, or a predicted condition of the subject being monitored, a body part thereof, and/or a plurality of body parts thereof and optionally functional interaction between a plurality of body parts.

As used herein, the terms "datum", "data", "parameter", "feature", and "condition" refer to, and are interchangeable with, the terms "predefined datum", "predefined data", "predefined parameter", "predefined feature", and "predefined condition", respectively, such that the type of "datum", "data", "parameter", "feature", or "condition" derived or predicted are known or are part of a known range of possible outcomes. According to some aspects, the predefined nature of a "datum", "data", "parameter", "feature", or "condition" refers to an anomaly being detected therein, in such cases, the anomaly property, in and of itself, in this case, is predefined.

As brought herein, the terms "conjoint" or "conjointly" in relation to the analysis of the EG signal, the PG signal, and the IG signal refer to performing analysis, such that the results of the analysis are based on all of the EG signal, the PG signal, and the IG signal inclusively and the exclusion of one of the EG signal, the PG signal, and the IG signal would not result in the same outcome of the analysis. For example, a conjoint analysis of the EG signal, the PG signal, and the IG signal has an outcome that is different from an analysis of any one or combination of two of the EG signal, the PG signal, and the IG signal. The difference in the outcome of a conjoint analysis of all of the EG signal, the PG signal, and the IG signal can be a difference in a confidence value, such as statistical significance, noise levels, or the like, and/or can be a difference in an actual value of the outcome of the analysis.

According to some embodiments, conjoint analysis can be achieved by inputting all of the EG signal, the PG signal, and the IG signal to a computational model for generating an outcome based on all of the EG signal, the PG signal, and the IG signal. Alternatively, according to some embodiments, one or more of the EG signal, the PG signal, and the IG signal are inputted to a first computational model, and the outcome thereof is inserted into another computational model(s) that receive as input at least some of the other signals, for deriving a datum based on all of the EG signal, the PG signal, and the IG signal. According to some embodiments, the other computational model(s) receive as an input outputs from earlier-stage computational models.

According to some embodiments, the conjoint analysis is based on additional inputs. According to some embodiments, the additional inputs include information related to the subject, such as age, gender, height, weight, clinical state, clinical history, medication, comorbidities, genetic information, or other demographic information. According to some embodiments, the additional inputs include signals obtained from additional sensors other than the IG sensor, EG sensor, or PG sensor. According to some embodiments, the additional inputs are provided from an external device or sensor, and/or by a user of the device, for example, via input means facilitated by the device.

As used herein, a computational model refers to a program executed/executable on a computer, configured to receive certain inputs, apply certain computations or algorithms, and generate at least one output. A computational model can be a machine learning algorithm, a filtering operation, a derivation model, a convolution model, or the like.

According to some aspects, examples of a datum derived from a conjoint analysis of all of the EG signal, the PG signal, and the IG signal include a datum related to cardiac phase annotation, such as Isovolumetric contraction, Ventricular contraction, Ventricular Ejection, Isovolumic relaxation, Ventricular filling, Diastasis, Artrial contraction, or the like. Advantageously, such a datum is usable for analyzing a cardiac function. According to some embodiments, the cardiac functions are usable for determining a clinical condition or pathologies related to the heart, such as hypertrophy, dilatation, reduced valve function, stenosis, sclerosis, insufficiencies, or the like.

According to some embodiment, the datum is usable for estimating a heart rate, for example in the range of 40 to 300 beats per minute, and is usable for rhythm analysis of the heart, detecting arrhythmias, stress detection, and identifying cardiac abnormalities. According to some embodiments, the heart rhythm analysis is indicative of a heart rhythm classifiable as normal, atrial fibrillation, supraventricular tachycardia, bradycardia, heart block, ventricular fibrillation, and the like, which are indicative of the functioning of the valves and identifying cardiac abnormalities.

According to some embodiments, the datum is usable for artificial valve function analysis, for example by providing an indication on one or more of valve timing, closure, leaflet stiffness, thrombosis, or the like, which may be advantageous for estimation of valve function for early intervention and therapy.

According to some embodiments, the datum is usable for stroke volume estimation, for example, in the range of 50 to 100 ml, which is indicative of the functioning of the valves and for identifying cardiac abnormalities.

According to some aspects, examples of a datum derived from a conjoint analysis of all of the EG signal, the PG signal, and the IG signal include a datum related to respiratory phase annotation, such as inspiration, end-inspiration, expiration, end-expiration, or the like. Advantageously, such a datum is usable as a basis for respiratory function analysis, the basis for compensating respiratory modulations in other bio-signals, and the basis for analyzing respiratory modulations in other bio-signals. According to some embodiment, the derived datum of the device is modulated based on respiratory activity.

According to some embodiments, the datum is usable for respiration rate estimation, for example, a respiration rate within the range of 4 to 40 breaths per minute, which is utilizable for rhythm analysis of the lung, identifying respiratory abnormalities, or the like.

As used herein, the term "sensor" refers to a module configured to sense or measure a designated property, particularly surface electrical potential differentials, surface vibrations, and impedance, wherein the sensing activity is performed directly by the sensor, for example when the sensor includes an interface with the body of the user, or indirectly, for example when the sensor is configured to be connected to an interface medium, such as an electrode, which is placed on the body of the user.

According to some embodiments, the interface medium includes an optical sensor, such as an optode, configured to measure changes in emitted, reflected, and/or absorbed electromagnetic waves at certain wavelengths.

According to some embodiments, one or more of the sensors in the device includes an interface medium configured to be placed on the body of the user, such as on a certain portion of the skin of the user.

According to some embodiments, one or more of the sensors includes a connector configured to achieve electrical, capacitive coupling, magnetic, and/or mechanical connection with an interface medium. Advantageously, such a configuration facilitates the use of replaceable interface media, such as replaceable electrodes for improving hygiene and/or measurement quality.

As used herein, the term "EG sensor" refers to a sensor configured to measure electrical potential differentials between two or more electrodes placed on the body of the user. An EG sensor can include a sensor functionally similar to Electromyography (EMG) sensors or Electrocardiography (EEG) sensors.

According to some aspects, the EG sensor includes, or is configured to be connected to, 2 electrodes, 3 electrodes, 4 electrodes, up to 10 electrodes, or more. According to some embodiments, the EG sensor includes, or is configured to be connected to an array of electrodes, which, advantageously, can facilitate vector analysis and/or surface potential mapping.

According to some aspects, the IG sensor includes, or is configured to be connected to, 2 electrodes, 3 electrodes, 4 electrodes, up to 10 electrodes, or more. According to some embodiments, the IG sensor includes, or is configured to be connected to an array of electrodes, which, advantageously, can facilitate vector analysis and/or surface potential mapping.

According to some embodiments, the IG sensor and the EG sensor are connected to shared electrodes. According to some embodiments, the shared EG/IG electrodes are utilized for acquiring EG and IG signals. According to some embodiments, the signals obtained from the shared EG/IG electrodes are frequency multiplexed, that is, the shared electrodes obtain EG signals at designated EG frequencies/frequency range and IG signals at designated IG frequencies/frequency range. According to some embodiments, the designated EG frequencies or a frequency range have lower frequencies than designated IG frequencies.

According to some embodiments, the designated EG frequencies or frequency ranges are from 0 Hz to 1 kHz while the designated IG frequencies or frequency ranges are above 1 kHz, above 10 kHz, above 20 kHz, or above 50 kHz, facilitating<aq< frequency multiplexing.

According to some embodiments, at least temporarily, there is an overlap between the designated EG frequency range and the designated IG frequency range, and a time multiplexing scheme is utilized for obtaining both EG signal and IG signals using shared electrodes, requiring sequential signal acquisition. For example, the designated EG frequency range is from 0 Hz to 1 kHz and the designated IG frequency range is from 0 kHz to 100 kHz or 200 kHz. According to some embodiments, time domain multiplexing is advantageous for facilitating bioimpedance spectroscopy (BIS).

According to some embodiments, all of the electrodes connected to or integrated with the IG sensor and the EG sensor are shared EG/IG electrodes. According to some embodiments, at least some of the electrodes connected to or integrated with the IG sensor and the EG sensor are shared EG/IG electrodes, while one or more of the electrodes connected to or integrated with the IG sensor or the EG sensor are dedicated electrodes.

As used herein, the term "IG sensor" refers to a sensor configured to measure conductivity properties between two or more electrodes placed on the body of the user, for example by providing a reference signal at one or more predetermined frequencies and for evaluating the impedance between two or more electrodes. According to some aspects, electrodes used for obtaining an EG signal or an IG signal are made of one or more of metal, carbon, cloth, foam, tab, and/or wet/gel electrodes. According to some embodiments, the type of electrode used for obtaining EG signals is determined based on the conditions of the measurements, for example, a cloth electrode can be used for longer-term wear, permitting normal skin respiration and stretching with movement, and a foam electrode could be suitable for short-term use and it is advantageous for providing high adhesion.

According to some embodiments, one or more of the electrodes in the EG or IG sensors can function as a reference electrode.

As used herein, the term "PG sensor" refers to a sensor configured to measure vibrations/auscultation, such as acoustic and/or mechanical vibrations caused by or resulting from the activity of one or more body parts of the user or by other factors, such as bodily fluids including blood, urine, stomach acid, salvia, or the like, non-bodily fluids including swallowed water or the like, non-bodily solids including swallowed food or the like, bodily gases including digestive byproducts or the like, non-bodily gases including air in the lungs or the like, artificial components such as implanted components, including prosthetic heart valves, joints, catheters, or the like.

According to some embodiments, the PG sensor includes, or is connected to, an interface configured to obtain, and optionally amplify, mechanical/acoustic vibrations/auscultation, such as a membrane, and to transform the obtained vibrations/auscultation to an electrical PG signal. According to some embodiments, the PG sensor is configured to transform the obtained vibrations/auscultation to an electrical PG signal using an accelerometer, a gyrometer, a magnetometer, a pressure-resistive sensor, a condenser sensor using a pressure-capacitor, such as a parallel-plate capacitor configured to convert acoustic pressure into displacement and to act as a moving plate of the capacitor, a piezoelectric sensor, a solid-state acoustic detector, microelectromechanical systems (MEMS) or the like.

According to some embodiments, the body part is an implanted artificial part and the PG sensor is configured to measure acoustic signals indicative of the state or condition of the body part. According to some embodiments, the body part is an implanted artificial part and the EG sensor and/or the IG sensor is configured to measure an EG signal and/or an IG signal indicative of an activity or a condition of the implanted artificial part.

The location of the device, particularly the location of the electrodes/interfaces, is selected based on the body part being measured. According to some embodiments, the electrodes/interfaces are configured to acquire the designated measurements noninvasively, that is, without penetrating the dermal tissue of the user. According to some embodiments, the electrodes/interface are minimally invasive to the dermal tissue of the user, for example, the electrodes/interfaces may include micro-needles or micro-needle grids.

According to some aspects, the device is placed on the front chest wall by the left side of the sternum at the 2nd, 3rd, and 4th ICS, or left midclavicular line at the 4th ICS, or the right side of the sternum at the 2nd ICS. Such placements are useful for facilitating cardiac analysis.

According to some aspects, the device is placed on the front or back chest wall on either side of the sternum, possibly between the clavicular rib and the last rib. Such placements are useful for facilitating lung analysis.

According to some aspects, the device is placed adjacent to a joint under examination, such that there is proper mechanical coupling predominantly to the PG sensor and for measuring the impedance for fluid and tissue analysis. In such uses, a strap is advantageous for holding the device in the desired position.

According to some aspects, the device is placed by the right or left lumbar region, umbilical region, right or left iliac region, or hypo-gastric region, for facilitating digestive analysis.

To hold the device in desired positions, self-adhesive electrodes can be advantageously used, in addition to or instead of the adhesion of the electrodes, the device can be positioned in place using a belt, lanyard, suction cup (with or without active vacuum), can be handheld in position, or be textile integrated, the device can include an adhesive member surrounding the electrodes and/or placed in spaces between the electrodes, or the device can be covered by plaster or other adhesive such that the device is at least partially or fully covered by the plaster, by applying a separate glue between device and skin, by using an additional double-sided adhesive as an interface, by placing an interface member on the skin which stays on the skin while the device can be attached to and removed from that interface, or the like.

As used herein, the term "indicative of" refers to the quality of the information derivable from the signal being affected by an activity or state of a body part being monitored. Particularly, a signal can be indicative of activities of multiple body parts since the monitored/target body part resides in the vicinity of other body parts and could be affected by the activities thereof. Additionally, even when the activity of the measured body part is not directly affected by the activities of other body parts, the activities of other body parts can affect the measured signal, for example, the electrical activity related to the movement of a skeletal muscle commonly affects the measurements of electrical potential differentials measured by EG electrodes configured to measure the electrical activity of the heart.

According to some embodiments, the device is a handheld device, configured to be briefly placed at a certain location, for a quick assessment or prediction of a condition.

According to some embodiments, the device includes a receptacle for mechanically connecting an external electronic device, such as a mobile device.

According to some embodiments, the device is wearable on the body of the user. According to some embodiments the device includes, or is configured to be attached to, an adhesive medium for attaching the device to the body of the user. According to some embodiments, the attachment of the device to the body of the user is achieved by an adhesion property of one or more interfaces/electrodes connected to, or integrated with, the device.

According to some embodiments, the device is attached/wearable to the body of the user using an attachment mechanism, such as a band that holds the device in a desired location/position by applying mechanical force against it in the direction of the body of the user.

According to some embodiments, the device is integrated into or is connectible to, a textile wearable by the user.

According to some embodiments, the body part is a biological body part. According to some aspects, the body part includes a heart, one or more of the lungs, an artery, a vein, and/or a valve.

As used herein, a body part refers to a tissue, a combination of tissues, an implanted tissue, an implanted device, an implanted artificial organ, or a combination thereof, in the body of the user. Commonly, the body part affects certain physiological functions such as cardiac functions, respiratory functions, neurological functions, digestive functions, vascular functions, structural functions, or the like.

According to some aspects, the device further includes a communication unit configured to obtain the EG signal from the EG sensor, the PG signal from the PG sensor, and the IG signal from the IG sensor, and to transmit said signals, or processed information derived from one or more of the signals, to an external communication unit.

According to some aspects, one or more of the signals transmitted to the external communication unit are processed at the device before transmission. The processing of the signal can include one or more of analog to digital conversions, digital-to-analog conversions, noise reduction, high-frequency filtration, low-frequency filtration, band filtration, modulation, adjusting an amplitude/gain of the signal, encryption, compression, dimension reduction, adaptive filtering, a machine learning model inference, or the like.

According to some embodiments, the device includes a signal processing unit, implemented in hardware, software, or a combination of hardware and software, configured to process one or more of the EG signal, PG signal, or IG signal.

According to some aspects, the device includes a memory configured for storing the acquired EG signal, PG signal, and IG signal. According to some aspects, the memory is a non-transitory memory. According to some embodiments, the memory is configured to store the signals at least until they are communicated with an external device wirelessly or via a wired connection. According to some embodiments, the memory is configured to store one or more of the signals in an unprocessed state, such as storing raw samples of the signal, or a processed state, such as a filtered, modulated signal, a machine learning model inference, or the like.

According to some aspects, the memory is configured for storing an output of the conjoint analysis or a result of an intermediary step thereof. According to some embodiments, the memory is a digital memory configured to store digital signals. According to some embodiments, the memory includes analog memory elements, configured to store analog signals.

As used herein, unless otherwise specified or implied, the term signal refers to either a digital signal of an analog signal, either in the time domain, in the frequency domain, or other domains such as z domain, s domain, or the like, either in a raw format or a transformation of the signal. According to some embodiments, a signal is transformed from the time domain to the frequency domain by performing a Fourier transform on the signal for example using a Fast Fourier Transform function. According to some embodiments, the signal undergoes transformation using one or more of the following transformation models/techniques: Fourier, cosine, wavelet, Hilbert, Laplace, Walsh-Hadamard, z, cepstrum, and/or Haar.

According to some embodiments, the device is configured to perform the conjoint analysis, for example, the device can be a standalone device configured to perform the analysis independently of external computational devices.

According to some embodiments, the device communicates the acquired EG signal, PG signal, and IG signal, or a pre-processed version thereof, to an external computational device, such as a server, a mobile device, a computer, a smart watch, another wearable electronic device, a base station, a relay device, or the like, wherein the external computational device is configured to obtain the EG signal, PG signal, and IG signal and perform the conjoint analysis, or relay the signals and/or processed information related to the signals or based on the signal to an additional external computational device for performing further processing, analysis, or display. In such a configuration, the device substantially performs the functions of signal acquisition while the external device performs all or most of the conjoint analysis. According to some embodiments, the pre-processed version of a signal or a plurality of signals includes an intermediary parameter extracted/derived from one or more of the signals.

According to some embodiments, pre-processing a signal includes applying normalization to the signal or to a derivative of a signal, for example, general normalization such that the signal is within a certain range of values, or normalization for preparing the signal to be fed as an input to a machine learning model.

According to some embodiments, normalization is performed within a certain segment or a certain phase of the signal. According to some embodiments, the normalization of one signal is performed independently of other signals. According to some embodiments, the normalization of one signal is performed based on, or in relation to, values of one or more of the other signals. According to some embodiments, normalization is applied to a feature extracted from one or more signals.

According to some embodiments, pre-processing a signal includes applying time domain adjustments, such as by applying dynamic time warping, stretching the signal in time, compressing the signal in time, and/or shifting the signal in time.

According to some embodiments, the relay device is a proprietary relay device, configured to facilitate secure communication with the device, for example by using a specific communication protocol, encrypted communication, or the like.

According to some embodiments, the device is configured to perform preliminary processing/analysis of the acquired signals and communicate intermediary analysis results to the external device for proceeding with the conjoint analysis.

According to some embodiments, the device is operable in mobile settings, such as in an ambulance, on a ship, on an airplane, or the like, or in a remote setting, such as at the home of the user.

According to some embodiments, the device is operable in clinical settings, such as for pre-OP, peri-OP, post-OP, or ICU settings, in an ambulatory setting, such as by a general practitioner, in care-setting, such as in an assisted living facility or a geriatric facility, in an emergency setting, such as in public places, in active settings, such as for sports medicine, in supporting and first responding settings, such as by fire fighters, the military, police force, or the like, in attachable doctor's office, such as in school-care, disaster relief, rural health, developing countries, correctional facilities, or the like.

As used herein, the term "OP" refers to an "operation" or another therapeutic procedure administered to the user, such as medication, invasive operation, or noninvasive intervention, such as an intervention using ultrasonic waves, magnetic fields, and/or electromagnetic waves or another medical intervention.

In mobile and remote settings, it is advantageous that a significant portion of the conjoint analysis is performed by an external device such as a server or a computer at the clinic of a caregiver or at a hospital.

According to some aspects, the device and the external device are connected via wires. According to some aspects, the device and the external device are connected wirelessly. According to some aspects, the device and the external device are connected via short-range communication, such as Bluetooth, Zigbee, wireless LAN, or the like. According to some aspects, the device and the external device are connected through a computer network. According to some aspects, the communication between the device and the external device is carried via a telecommunication infrastructure. According to some embodiments, at least some of the conjoint analysis is performed using a cloud-computing system.

According to some embodiments, the device is configured to be used in a clinical setting, such as at a doctor's clinic or in a hospital. In such cases, the device can be connected via wires, or wirelessly, to the external device. According to some embodiments, the device is further configured to be powered by the external device, that is, the device is configured to receive power for performing the operation thereof from the external device, for example, through the wire connection with the external device.

According to some embodiments, the device is configured to be battery-operated. According to some embodiments, the battery is a rechargeable battery. According to some embodiments, the battery is a replaceable battery. According to some embodiments, the device is powered by a wireless power delivery. According to some embodiments, the device is powered using an energy harvesting mechanism, such as a mechanical energy harvesting mechanism, electromagnetic energy harvesting mechanism, thermal energy harvesting mechanism, or the like, wherein the harvested energy is obtained from the body of the subject and/or the environment of the device. According to some embodiments, the energy harvesting mechanism is based on radio waves, body movement, body temperature, ambient light, radio waves, body fluids, changes in pressure, ambient radiation, fluid or gas flow, or the like.

According to some embodiments, the external device includes one or more of a phone, a computer, a tablet, a smartwatch, a server, a cloud-computing system, or the like.

According to some aspects, the wearable device further includes a processor configured to obtain the EG signal from the EG sensor, obtain the PG signal from the PG sensor, obtain the IG signal from the IG sensor, and conjointly analyze the EG signal, the PG signal, and the IG signal to allow for an assessment of a predefined condition of the body part or the subject by deriving at least one datum based on all of the EG signal, the PG signal, and the IG signal.

According to some aspects, deriving at least one datum based on all of the EG signal, the PG signal, and the IG signal includes detecting a feature preferably including at least one of a distance between two predefined points of interest, a rate of occurrence of an event, or a value at a point of interest.

According to some aspects, deriving at least one datum based on all of the EG signal, the PG signal, and the IG signal includes detecting a predefined feature preferably including at least one of a distance between two predefined points of interest, a rate of occurrence of an event, or a value at a point of interest.

As used herein, the terms "feature" of "predefined feature" refers to a property or a combination of properties derived from one or more of the signals either in isolation, by regarding a signal without considering other signals, or in the combination of two or more of the signals.

A predefined feature includes, for example, a property of a signal related to the intensity thereof at certain points, locations of local or global maxima/minima, differences between values of two or more points, rate of change, a statistical property, or the like in the signal/signals, or an associated signal or signals derived therefrom, such as a time derivation of the signal in the first order, second order, or higher order. Such features can be obtained from the signal or associated signals derived therefrom in the time domain or in the frequency domain.

According to some embodiments, a predefined feature is derived using transformations or coefficients of transformations of one or more of the signals, statistical moments, extrema, percentiles, model fits, and coefficients of model fits, such as polynomial, exponential, geometric, distributions, series, rational, spline, autoregressive, or the like, signal morphology such as the rise and/or fall-times, areas, inter- and intra-signal segmentation, area ratios between a rising and a falling portion of signals, for instance, threshold crossings, amplitudes of characteristic points, ratios and norms thereof, or the like.

According to some embodiments, a predefined feature is derived using analysis and models based on complexity/entropy features, such as fractal dimension, approximate entropy, sample entropy, conditional entropy, fuzzy, or the like.

According to some embodiments, a predefined feature is derived using analysis and models based on time series analysis, such as long-range correlations, correlations, and templates.

According to some embodiments, a predefined feature is derived using analysis and models based on another one or more features.

According to some aspects, a convolution model is utilized for deriving certain features from a signal or from a combination of signals.

The conjoint analysis of the EG signal, the PG signal, and the IG signal is performed and is configured to allow for an assessment of a predefined condition of the body part or the subject. The wording "allow for an assessment" relates to the nature of the extracted/derived datum/data as being associated with, relevant to, and/or indicative of the predefined condition.

As used herein, the term "predefined condition" refers to either a present/existing/prevailing state or condition of the body part, such as a present state or condition of a heart, or to a projected/predicted state of the body part at a future point, following a certain time period and/or following a certain planned procedure, such as a diagnostic or therapeutic procedure.

According to some aspects, a predefined condition is a predicted reaction or state of the body part in case a therapeutic procedure is administered to the user, such as medication, invasive operation, or noninvasive intervention, such as an intervention using ultrasonic waves, magnetic fields, and/or electromagnetic waves. According to some embodiments, the term "therapeutic procedure" can refer to one or more of physio therapy, psychotherapy, or other forms of therapy.

According to some aspects, an existing or suspected predefined condition relates to a clinical/pathological condition of the user or of the body part.

According to some aspects, the predefined condition could relate to both an existing condition and a predicted condition. For example, a predefined condition could relate to a detection of a pathological condition of the body part and to a predicted progression thereof. For example, a predefined condition could be related to a detection of a pathological condition of the body part and to a predicted reaction of the body part or of the user to one or more interventions or avoidance/lack of one or more interventions.

According to some aspects, the predefined condition is an assessment and/or prediction of the general state or fitness of the user.

According to some embodiments, the predicted predefined condition includes an estimation of a pre-op or a post-op risk, including for example peri- and post-op morbidity and mortality, shock, hemorrhage, wound infection, deep vein thrombosis, pulmonary embolism, pulmonary embolism, pulmonary complications, urinary retention, heart failure, myocardial infarction, or the like. The condition is utilizable for assessing a patient's status pre-OP to decide whether the OP risk is acceptable, or if pre-OP rehab is needed, what pre-, peri-, and post-OP path is appropriate for the patient, or the like. According to some embodiments, a pre-OP use can relate to the prediction of post-OP complication in the context of pre-OP risk evaluation, for example, the probability of 30-day mortality or morbidity.

According to some embodiments, a pre-OP application of a device on a patient/subject involves the steps of acquiring IG, PG, and EG sensor data/signals, extracting features therefrom, inputting the extracted features into the pre-learned or trained model, receiving a risk score or probability of a general adverse event post-OP, optionally, with a confidence interval or a probability of specific adverse outcome post-OP with confidence interval.

According to some embodiments, the device is utilized for indicating a cardiac condition, such as stenosis, infarct, valve insufficiency, or the like, wherein the device is applied to the patient for acquiring IG, PG, EG sensor data, extracting features, inputting the extracted features into pre-learned/trained model, receiving a yes/no answer or probability, preferably with a confidence interval, if the heart is healthy, and/or receiving a probability, preferably with a confidence interval, for specific pathologies to be present.

According to some embodiments, the device is utilized for indicating a lung/respiratory condition, such as COPD, acute infection, or the like, wherein the device is applied to the patient for acquiring IG, PG, and EG sensor data, extracting features, inputting features into pre-learned/trained model, receiving a yes/no answer or a probability with a confidence interval and/or a stage estimation and functional status of the lungs.

According to some embodiments, the device is utilized for estimating a functional state/status of an artificial heart valve, such as correct closure or leakage, by applying the device to the patient/user, acquiring IG, PG, and EG sensor data, extracting features, inputting features into pre-learned/trained model, receiving estimations on the status of the heart valve including leaflet flexibility, leakage, closing characteristics, forming of thrombosis, or the like.

According to some embodiments, the device is utilized for indicating or predicting a failure of an artificial heart valve, for example, stiffening of leaflets, thrombotic events, or the like, by repeatedly applying the device to the patient/user, acquiring IG, PG, and EG sensor data, extracting features, inputting features into pre-learned/trained model, and obtaining wear estimates and projecting a trajectory of future wear, possible failure or functional impairment using known early changes in the data.

According to some embodiments, the device is utilized for estimating a post-intervention trajectory of the user acquiring IG, PG, and EG sensor data, extracting features, and inputting features into the pre-learned model, possibly together with additional patient information, such as age, gender, height, weight, medication, known comorbidities, or the like, to estimate possible outcomes of an intervention prior to its application.

According to some embodiments, the device is utilized for generating an individualized therapy, for example, after a cardiac event, during COPD monitoring, or the like, by applying the device once or regularly after an adverse event, after diagnosis of a chronic disease, or after an intervention to track the disease or therapy success or adjust the therapy specifically to the needs of the patient/user based on the data acquired by the system.

According to some embodiments, the processor is configured to detect at least one point of interest or a segment in one signal based on another detected point of interest or segment in another signal. According to some embodiment, the processor is configured to detect a segment in one signal and to use the detected segment to indicate a region of interest in another signal for detecting a point of interest therein.

According to some embodiments, the first point of interest or a first segment is detected in a first signal, based on which, the second point of interest or a second segment is detected in a second signal, based on which, the third point of interest or a third segment is detected in a third signal.

According to some embodiments, a point of interest in the EG signal is the R-peak. This point can be detected by identifying the point with maximum absolute amplitude in a segment between prominent absolute signal derivative values.

According to some embodiments, a point of interest in the PG signal is the S1 starting point. It can be detected by identifying the onset of a segment with high signal energy in the frequency range of the S1 heart sound.

According to some embodiments, a segment of interest in the PG signal is the S3 sound segment, which is caused by a low-frequency brief vibration occurring in the early diastole at the end of the rapid diastolic filling period of the right or left ventricle. According to some embodiments, the S3 segment is detected by identifying the onset and the offset of a segment with high signal energy in a frequency range associated with the S3 heart sound.

According to some embodiments, a segment of interest in the PG signal is the S4 sound segment, which, if present, is caused by atrial contraction. According to some embodiments, the S4 sound segment is detected by identifying the onset and the offset of a segment with high signal energy in a frequency range associated with the S4 heart sound.

According to some embodiments, a segment of interest is a cardiac cycle in the EG signal. This segment can be detected by identifying the R-peaks of two successive heart cycles.

According to some embodiments, the processor is configured to detect two or more points of interest in the same signal, for example, the processor is configured to detect two or more maxima and/or minima in a signal.

As used herein, unless otherwise specified, the term "point of interest", refers to a "predefined point of interest", such that it refers to a point of particular known clinical interest and/or a point of a specific statistical or computational interest.

As used herein, unless otherwise specified, the terms "segment", "section", and "phase" are interchangeably used and refer to a particular time range, frequency range, or another one-dimensional or multi-dimensional section in the signal or a transformation thereof, and the terms are interchangeable with the terms "predefined segment", "predefined section", and "predefined phase", respectively.

The term "predefined" refers to a segment, section, phase, point, condition, and/or feature being of a clinical and/or computational significance, either a known significance, such as a medically defined point in a signal relating to a known activity or property of the body part, or an unknown significance, such as a feature extracted/derived by a computational model internally with or without being presented to a user or operator, such as features extracted/derived by a convolution layer in a machine learning model.

According to some aspects, a "predefined" property refers to a classification of one or more of a plurality of known classes. According to some aspects, the predefined property includes a predicted trajectory or predicted trajectories of one or more of the signals in a time following the measurements, such as a projected corridor or potential predicted values.

The term "predefined" also includes the property of a segment, section, phase, point, condition, and/or feature being anomalous and not conforming to expected values or properties. In this case, the anomaly itself is considered predefined.

According to some aspects, the conjoint analysis includes performing time synchronization between the different signals. According to some aspects, time synchronization is a sub-millisecond synchronization. According to some aspects, time synchronization of the signal is performed by time-stamping each of the signals at a predetermined frequency signal and utilizing interpolation schemes for aligning the timing of the different signals. According to some embodiments, the predetermined frequency signal has frequencies higher than 1 kHz. According to some embodiments, synchronization is performed by synchronizing sampling events of the signal acquisition across or between two or more of the signals.

According to some aspects, the conjoint analysis includes performing point and/or segment detection based on one or more of the EG signal, the PG signal, or the IG signal to detect a point and/or a segment of interest and analyze the other signals based on the detected point or segment of interest.

According to some aspects, performing segment detection includes segmenting the signal to predefined sections or predefined phases of a predefined biological event, for example, a respiratory cycle, a cardiac cycle, and/or a circadian cycle, or another biological event, voluntary or involuntary.

According to some aspects, analyzing the other signals based on the segmentation includes identifying characteristic properties in one or more of the other signals based on the detected point and/or segment.

As used herein, the term "biological event" refers to an activity affected in the body of the user, either voluntarily or involuntarily, by the body part being monitored or by other body parts.

In some respects, the biological event is a repeated cyclic/rhythmic event, such as a respiratory or a cardiac cycle or part thereof. The terms cyclic or rhythmic refer to the repeated nature or patterned nature of the event and/or to a sequential property thereof, for example, an expected sequence of phases in a cardiac cycle, an expected sequence of a respiratory cycle, an expected sequence of a circadian cycle, or the like, that commonly form a pattern associated with the activity. Such a repeated event can be involuntary, for example, a cardiac cycle or an involuntary respiratory cycle, or at least partially voluntary, such as guided respiration activity.

In some aspects, a biological event is a non-cyclic event, such as skeletal muscle movement, coughing, swallowing, blinking, digestive-related activity, or the like.

According to some embodiments, a model or a plurality of models are employed for detecting a point of interest or a segment, such as a point detection model or a segmentation model. According to some embodiments, one or more of the models corresponds to a machine learning model.

According to some embodiments, the derivation of at least one datum based on all of the EG signal, the PG signal, and the IG signal in a conjoint analysis is performed using a machine-learning model. According to some embodiments, the machine learning model is a supervised machine learning model, trained based on labeled input and output training data sets. According to some embodiments, the machine learning model is an unsupervised machine learning model, trained based on unlabeled or raw data.

According to some embodiments, a machine-learning model is trained on the device, for example, based on acquired signals and/or data from a subject.

According to some embodiments, the derivation of at least one datum based on all of the EG signal, the PG signal, and the IG signal in a conjoint analysis is performed by inputting all of the EG signal, the PG signal, and the IG signal to a single model for deriving the datum.

According to some embodiments, the derivation of at least one datum based on all of the EG signal, the PG signal, and the IG signal in a conjoint analysis is performed by inputting the EG signal, the PG signal, and the IG signal to a plurality of models for deriving the datum, wherein an output of at least one model is inserted as an input to another model, forming a multi-stage derivation. According to some embodiments, in a multi-stage derivation, information, such as segments, features, and/or points, in one signal, using one model is derived based on derived information, such as segments, features, and/or points, in another signal, using another model.

According to some embodiments, a segment detected in one signal is used for detecting a point of interest in another signal. According to some embodiments, a point of interest detected in one signal is used for detecting a segment in another signal. According to some embodiments, a point of interest detected in one signal is used for detecting another point of interest in another signal. According to some embodiments, a segment detected in one signal is used for detecting another segment in another signal.

According to some embodiments related to cardiac monitoring, an R is detected in an EG signal, based on which, an S1 is detected in a PG signal, based on which a B is detected in an IG signal. The parameter "R" in an EG signal refers to the R wave in a QRS complex and represents the electrical stimulus as it passes through the main portion of the ventricular walls, the parameter "S1" in a PG signal refers to a first heart sound amplitude/energy, and the parameter "B" in an IG signal represents the opening of the aortic valve.

According to some embodiments, a point of interest in an EG signal includes one or more of a Q point, an R point, an S point, a J point, a T point, and/or a U point.

According to some embodiment, a section, or a section of interest in an EG signal includes one or more of a PR section, a P wave section, a QRS section, an RR section, an ST section, a TO section, a TP section, a QT section, or any section between two points of interest in an EG signal.

According to some embodiments, a point of interest in an IG signal includes one or more of an A point, a B point, a C point, an X point, a Y point, and/or an O point.

According to some embodiment, a section, or a section of interest in an IG signal includes one or more sections of any section defined by two points of interest in an IG signal.

According to some embodiments, the EG signal is used for detecting an R-Peak or a sharp R-Peak point, and, using the R-Peak as a landmark, the S1 point in the PG can be detected in noisy environments as well. In an exemplary implementation, the S1 point is detected using a probability and distribution-based approach using the R-Peak as a reference point in time. In addition, the T-wave extracted from the EG signal can be used to define a time segment or boundary as well, further enhancing the robustness of the detection of S1 by providing a plausibility window. According to some embodiments, once the R and S1 are identified, the B-Point in the cardiac IG curve is identified based on the R and S1, for example using distribution approaches based on the timing between R-Peak, S1 peak, and B-point, advantageously enabling the detection of the B-point even when the IG signal is weak or the signal to noise ratio thereof is low.

According to some embodiments, an R-peak point is detected using the EG signal, then a B-point and/or a C-point are detected from the IG signal. Exemplary, the PG signal is utilized for determining sections of interest, enhancing the accuracy of measurements of other signals, and/or determining the quality of the EG and/or IG signals.

According to some embodiments, in the conjoint analysis, the P point, Q point, R point, S point, and/or T point are identified/detected from the EG signal, the S1 point and/or S2 point are detected from the PG signal, and the A point, B point, C point, V point, X point, Y point, and/or O point are detected from the IG signal. According to some embodiments, the cardiac cycle is segmented at least into the following two phases: ventricular systole (the contraction phase) and ventricular diastole (the relaxation phase). According to some embodiments, the segment of isovolumetric contraction (also known as the pre-ejection period) is identified between the EG signal Q-Point and the IG signal B-point. According to some embodiments, a segment is identified beginning at the IG signal B-point and ending at the S2 onset in the PG signal marking the ventricular ejection (also known as left-ventricular ejection time). According to some embodiments, a segment is identified beginning at the EG signal Q-point and ending at the IG X-point depicting the total systolic time. According to some embodiments, a ventricular isovolumetric relaxation segment is identified starting at the PG signal S2 onset (closing of the aortic valve) and ending at the IG signal O-point (opening of the mitral valve). According to some embodiments, a segment is identified demarking the atrial systole between the EG signal P-Point (atrial depolarization) and the PG signal S2-onset.

According to some embodiments, points of interest, such as peaks, troughs, and zero crossings in a respiratory signal are used for identifying phases of inspiration, such as end-inspiratory, expiratory, and end-expiratory segments.

In certain situations, especially in signals acquired with added noise, for example, due to interferences or activity of the user or their surrounding, the detection of an S1 onset in the PG signal is challenging. According to some embodiments, in the EG signal, the R-peak corresponds to the depolarization of the ventricles that causes contraction of the large ventricular muscles, while the C-point in the IG corresponds to the maximum ventricular contraction. Because the S1 onset corresponds to the closing of the mitral valve, it resides, timewise, between the R-peak in the EG and the C-point in the IG, therefore, detecting R and C points is more robust than detecting S1 in high noise scenarios, due to the high-frequency content and large amplitudes. Accordingly, in some embodiments, when R and C are identified, the segment defined between them is specifically searched for the S1 onset, which facilitates a much more robust detection thereof.

According to some embodiments, since an A-point of an IG signal follows a P-point and precedes S1 onset, a local minimum in the IG signal in a segment from EG signal P-point to PG signal S1 onset is used to identify an IG signal A-point.

The X point in the IG signal and the S2 onset point in the PG signal are adjacent in the time domain since the IG signal X point relates to the stop of blood flow caused by the closing of the aortic valve, which also generates the S2 sound. However, both the X point in the IG signal and the S2 onset point in the PG signal may not be easily detectable in a high-noise environment. Advantageously, according to some embodiments, the positions of the X point in the IG signal and the S2 onset point in the PG signal are estimated by using the C to P segment as well as probabilistic position information within this segment. In addition, according to some embodiments, the close relation between the X point in the IG signal and the S2 onset point in the PG signal is used to increase the robustness of detection of the exact location by exploiting their redundant position information.

According to some embodiments, a segment of interest is identified using a Q point, marking the beginning of ventricular depolarization, and an X point marks the closing of the aortic valve, Defining a Q-X segment. According to some embodiments, an S1 sound/PG segment is identified based on the Q-X segment, particularly within the time frame defined by the Q-X segment.

According to some embodiments, the conjoint analysis of the signals is utilized by determining the heart rate derived from the EG signal and the heart rate derived from the IG signal combinedly for evaluating higher reliability of heart rate measurement.

According to some embodiments, the heart rate derived from the EG signal, the heart rate of the IG signal, and the heart rate of the PG signal are conjointly analyzed for estimating a heart rate with high reliability.

According to some embodiments, using the time difference between successive EG R-peaks, a beat-to-beat EG heart rate time series is derived, and a second heart rate time series is calculated from successive IG C-points. According to some embodiments, both heart rate time series are modulated by respiratory sinus arrhythmia and thus are conjointly used for respiratory rate estimation.

According to some embodiments, the time difference between successive EG R-peaks is used for deriving a beat-to-beat EG heart rate time series, a second heart rate time series is calculated from successive IG C-points, and a third heart rate time series is calculated from successive S1 points as well as successive S2 points, wherein all three heart rate time series are modulated by respiratory sinus arrhythmia and thus conjointly used for a respiratory rate estimation.

According to some embodiments, the left ventricular ejection time (LVET) is derived from the IG signal and PG signal between the B-point and S1 onset. According to some embodiments, the pre-ejection period (PEP) is measured from the EG signal Q-point to the IG signal B-point. Consequently, according to some embodiments, the total systolic time (TST) is calculated as the sum of LVET and PEP.

According to some embodiments, the device is configured to be used for long-term monitoring of the user, for example, the device is configured to be worn or applied to the user for periods extending beyond 6 hours.

Advantageously, long-term monitoring could be indicative of events that occur scarcely or could be indicative of a trend in a detected condition. For example, long-term monitoring could be used after an operation or treatment procedure to detect a possible failure following the procedure and/or for detecting a recovery process following the procedure. In other examples, long-term monitoring could be used for home monitoring to provide long-term information for a caregiver on the condition of the user, for example, for long-term follow-ups.

According to some embodiments, the device is configured to be used for medium-term monitoring of the user, for example, the device is configured to be worn or applied to the user for periods ranging from 10 minutes to 6 hours. According to some embodiments, the device is used for short-term monitoring, for example, the device is configured to be worn or applied to the user for periods ranging from 1 second to 10 minutes.

Advantageously, medium-term and short-term monitoring could be used for assessing the condition of the user before or after a treatment procedure, for usage in an ambulance setting, for general ward monitoring, or the like.

According to some embodiments, the device is configured to be used periodically, for example, once every hour, once a day, once every predefined period of time, or once every predefined event, such as follow-up visits to a clinic.

According to some embodiments, information obtained in one or more previous uses of the device is utilized for deriving the at least one datum. According to some embodiments, the at least one datum is indicative of a change in the condition of the user or of the body part by detecting a change in information or values between different uses.

According to some aspects, the conjoint analysis includes deriving data from each of the signals, wherein the data derived from different signals include redundancy therebetween. According to some aspects, the redundancy in the data derived from different signals includes deriving data, such as a value, of the same parameter, such as a heart rate, multiple times, wherein each time a different signal or combination of signals is used for deriving the data.

For example, the redundancy in data could be achieved by determining a heart rate based on the PG signal, a heart rate base on the IG signal, and a heart rate based on the EG signal.

According to some aspects, the conjoint analysis further includes estimating a signal quality or a confidence parameter/indicator of a selected one of the EG signal, the PG signal, or the IG signal by comparing a datum derived therefrom with a datum derived from one or more of the other signals as reference signals. According to some aspects, determining a reference timing datum from the reference signals, estimating a signal quality or a confidence parameter/indicator of a selected one of the EG signal, the PG signal, or the IG signal is achieved by determining a timing datum of the selected signal and comparing the timing datum of the selected signal with the reference timing datum from the reference signals.

According to some aspects, determining a reference timing datum from the reference signals, estimating a signal quality or a confidence parameter/indicator of a selected one of the EG signal, the PG signal, or the IG signal is achieved by estimating a first datum based on a physiological parameter from the reference signals, estimating a second datum based on the same physiological parameter from the selected signal, and determining a discrepancy value between the datum estimated from the selected signal and the datum estimated from the reference signals.

As used herein, the terms confidence parameter or confidence indicator refer to an indicator related to how certain is the model with regards to the determined or predicted condition or datum. In some embodiments, the confidence parameter corresponds to a confidence interval, such that the confidence, in the statistical sense, relates to the probability or certainty in that the detected or predicted value is correct or that the measured value corresponds to the actual value. For example, a confidence interval with a 95% confidence level indicates that the actual value will be in a 95% certainty within the upper and lower values specified by the confidence interval. In some embodiments, the estimated signal quality or confidence parameter indicate a noise level or a distribution of possible values around the measured, derived, or predicted value or within a range including the measured, derived, or predicted value.

According to some embodiments, a heart rate derived from the EG is used as a reference value for heart rates derived from the IG and PG signals, such that, if the IG or PG heart rate differs from the EG heart rate, a high difference is indicative a low signal quality in these signals and thus the confidence in the features derived thereof is reduced.

According to some embodiments, an inter-signal correlation is modeled, for example, by utilizing transfer functions. According to some embodiments, the IG signal is estimated from the EG signal. An estimation error, based, for example, on the difference between the measured IG signal and the estimated IG signal, is indicative of the signal quality.

According to some embodiments, self-similarity of periodic signals, for example, heart cycle segments within the signals, are used for estimating the signal quality. Such that, for example, if a signal segment in one or signals corresponding to a periodic segment is similar, for example, in signal values of features, to preceding and/or succeeding periodic segments in that signal(s), a high signal quality is indicated. If, on the other hand, a signal segment differs from preceding and/or succeeding periodic signal segments, this indicates a bad signal quality and thus reduced confidence.

According to some aspects, the conjoint analysis includes determining a first physiological parameter from one or more of the EG signal, the PG signal, or the IG signal, determining a second physiological parameter from the other one or more of the EG signal, the PG signal, or the IG signal, and deriving a third physiological parameter based on the first physiological parameter and the second physiological parameter. According to some aspects, the first parameter, the second parameter, and the third parameter are different from one another. According to some aspects, the first parameter, the second parameter, and the third parameter are the same parameter. According to some aspects, the first parameter and the second parameter are the same parameters, and the third parameter is a different parameter.

As used herein, the term "physiological parameter" is interchangeably used with the terms "bodily parameter" and "biological parameter", and refers to parameters indicative of a physiological state or activity of the user or body parts of the user, such as blood pressure, body temperature, breathing rate, heart rate, blood oxygen saturation, and various electrophysiological signals, representing, at least partially, the operation of the body of the subject, and such parameters are indicative or usable for the evaluation or monitoring of the health state of the user.

According to some aspects, a feature derived from one of the signals is derived based on another feature derived from another signal.

According to some aspects, a feature corresponds to a value corresponding to a characteristic of the signal. According to some aspects, a feature is provided as an input to an input layer of a machine learning model, such as an input layer of a neural network.

According to some aspects, the conjoint analysis further includes providing the derived plurality of features to a detection and/or prediction model, wherein the detection and/or prediction model preferably corresponds to a machine-learning model, and generating, from the detection and/or prediction model, a score or a value indicative of a probability of the predefined condition of the body part.

According to some embodiments, one or more of the models include a supervised machine-learning model, such as random forest, XGBoost, logistic regression, artificial neural networks, recurrent networks, deep learning models, probabilistic models, statistical models, Bayesian models, support vector machines, and/or kernel methods.

According to some embodiments, one or more of the models is an anomaly detection model, such as replicator neural networks, autoencoders, variational autoencoders, long short-term memory neural networks, hidden Markov models, and/or fuzzy logic models.

According to some embodiments, one or more of the models includes a clustering model, such as centroid models, distribution models, density models, subspace models, group models, and/or graph models.

According to some embodiments, one or more of the models includes a pattern recognition model, for example, models using classification, clustering, ensemble, and/or regression methods.

According to some embodiments, one or more of the models are trained using anonymized training sets.

According to some embodiments, the system is configured to detect a specific cardiac pathology, for example, aortic stenosis. According to some embodiments, in a training phase of one or more of the computational models applied in the device or the system, the computational model is applied to a number of patients with a known indication(s) of aortic stenosis as well as patients without that indication, generating an annotated dataset including at least EG, IG, and PG data as well as the annotation of the presence or absence of aortic stenosis. The EG, IG, and PG data are then pre-processed and cleaned, which includes filtering an artifact handling from movements as well as cleaning cross-talk between different bio-signals. According to some embodiments, using conjoint signal analysis, segmentation, and point detection is performed to identify characteristic points and segments in the signal such as QRS complex, heart sounds, respiratory phases, and phases in the cardiac cycle. From the identified points and segments, one or more intra- and inter-signal features such as amplitudes, distances, transformations, and coefficients are extracted, which are then fed into a supervised machine learning algorithm alongside the annotations of the presence or absence of aortic stenosis, thereby creating a model representing the relation between the features from the EG, IG, and PG signals as well as the outcomes. Optionally, according to some embodiments, additional patient information, such as one or more of age, gender, height, weight, comorbidities, medication, previous interventions, and the like, can be used as inputs. When the device/system is applied for a new user/patient, EG, IG, and PG signals are measured/recorded and the same signal processing and feature extraction methods as in the training phase are applied. When presenting those features and the optional, additional features to the pre-learned/trained model, a result will be generated through inference which is interpreted as the presence or absence of aortic stenosis within a certain confidence interval.

According to some embodiments, the signals, features, and optional patient information are combined with a set of post-operative, 30-day outcomes such as heart failure, stroke, surgical site infections, and the like in the training phase. According to some embodiments, in the inference phase, the system is applied pre-operatively to the patient in order to assess the risk of the intervention by providing a risk score or specific probabilities of adverse outcomes.

According to some aspects, there is provided a system for measuring and/or monitoring biological signals of the body part of a subject, corresponding to the appended claims. The system includes the wearable device according to the aspects brought above, a system communication unit adapted to communicate with the communication unit of the wearable device to obtain the EG signal, the PG signal, and the IG signal therefrom, and a processor. The processor is configured to obtain the EG signal, the PG signal, and the IG signal from the system communication unit, and conjointly analyze the EG signal, the PG signal, and the IG signal to allow for an assessment of a predefined condition of the body part by deriving at least one datum based on all of the EG signal, the PG signal, and the IG signal, preferably, wherein deriving at least one datum based on all of the EG signal, the PG signal, and the IG signal comprises detecting a feature preferably including at least one of a distance between two predefined points of interest, a rate of occurrence of an event, or a value at a point of interest.

According to some aspects, the conjoint analysis performed by the processor in the system corresponds to the conjoint analysis elaborated in relation to the aspects provided above.

According to some aspects, there is provided a system for measuring and/or monitoring biological signals of the body part of a subject, corresponding to the appended claims. The system includes a processor and a device. The device includes a surface electrical potential differentials sensor, electro-gram, EG, configured to measure the electrical activity of the body part and to provide at least one EG signal indicative of the electrical activity of the body part, a surface vibrations sensor, phono-gram PG, configured to measure an acoustic activity of the body part and to provide a PG signal indicative of the acoustic activity of the body part, and an impedance sensor, impedance-gram, IG, configured to measure an electrical impedance of the body part and to provide an IG signal indicative of the electrical impedance of the body part and a communication circuit, configured to transmit the signals from the EG sensor, the PG sensor, and the IG sensor to the processor. According to some aspects, the processor is configured to obtain the EG signal, the PG signal, and the IG signal from the communication circuit, and conjointly analyze the EG signal, the PG signal, and the IG signal to allow for an assessment of a predefined condition of the heart by deriving at least one datum based on all of the EG signal, the PG signal, and the IG signal,

According to some aspects, the conjoint analysis performed by the processor in the system corresponds to the conjoint analysis elaborated in relation to the aspects provided above.

According to some embodiments, the system includes additional sensors. According to some embodiments, the sensors are embedded in the device. According to some embodiments, the sensors are not embedded in the device.

According to some embodiments, the additional sensors include one or more of a body temperature sensor, an ambient temperature sensor, an oximeter, a glucose sensor, a volatile compounds sensor, a barometer, an accelerometer, a plethysmograph, or the like.

According to some aspects, the conjoint analysis further includes deriving a datum based on all of the EG signal, the PG signal, and the IG signal, as well as based on one or more of the signals obtained by the additional sensors.

According to some embodiments, the additional sensors include one or more inertial measurement unit (IMU) sensors, such as an accelerometer, a gyro-meter, a magnetometer, or the like, is used in the device, advantageously facilitating one or more of a detection of motion and thus motion artifact handling and/or compensation, providing additional input for respiratory signal estimation for example based on chest movement as a respiratory signal, providing an additional input for cardiac diagnostics for example by using a ballistocardiogram or a gyro-cardiogram, providing additional input for joint analysis such as movement angles, distance traveled, rotation angles, or the like, providing an estimation of activity such as steps, energy expenditure, posture, or the like, facilitating cardiac and respiratory analysis in combination with activity, providing posture estimation for posture-dependent signal analysis.

According to some embodiments, the additional sensors include one or more photoplethysmography (PPG) sensors, such as pulse wave, tissue oxygenation, and perfusion, which are advantageous to the conjoint analysis by indicating pulse arrival time which is usable for determining a distance between R-Peak and PPG onset, and can be used to estimate cardiac timing parameters, vessel stiffness, and can give insight into hemodynamic parameters, hemorrhage, or the like. Additionally, the PPG sensors are usable for estimating local tissue oxygenation as a proxy for oxygen delivery, and tissue perfusion is usable as an indicator for circulatory parameters.

According to some embodiments, the additional sensors include one temperature sensors, such as a body temperature sensor, the signals of which are indicative of systemic effects, such as inflammation or fever. Additionally, the temperature measurements are usable for peri-OP temperature management of the patient/user.

Certain embodiments of the present disclosure may include some, all, or none of the above advantages. One or more technical advantages may be readily apparent to those skilled in the art from the figures, descriptions, and claims included herein. Moreover, while specific advantages have been enumerated above, various embodiments may include all, some, or none of the enumerated advantages.

In addition to the exemplary aspects and embodiments described above, further aspects and embodiments will become apparent by reference to the figures and by the study of the following detailed descriptions.

### Brief Description of the Drawings

Examples illustrative of embodiments are described below with reference to the figures attached hereto. In the figures, identical structures, elements, or parts that appear in more than one figure are generally labeled with the same numeral in all the figures in which they appear. Alternatively, elements or parts that appear in more than one figure may be labeled with different numerals in the different figures in which they appear. Dimensions of components and features shown in the figures are generally chosen for convenience and clarity of presentation and are not necessarily shown in scale. The figures are listed below.
Fig. 1 schematically illustrates a device, according to some embodiments;
Fig. 2 schematically illustrates a device in communication with an external device, according to some embodiments;
Fig. 3 schematically illustrates a device with an integrated processor, according to some embodiments;
Fig. 4 schematically illustrates a system including a device operable in a mobile setting, according to some embodiments;
Fig. 5 schematically illustrates a patch with interfaces, according to some embodiments;
Fig. 6a schematically illustrates a wearable system including a device and a strap, according to some embodiments;
Fig. 6b schematically illustrates a wearable system including a device attached to a shirt, according to some embodiments;
Fig. 7a schematically illustrates a handheld apparatus, according to some embodiments;
Fig. 7b schematically illustrates a system including a device with a docking receptacle for a mobile device, according to some embodiments;
Fig. 8 schematically illustrates a method for measuring and/or monitoring biological signals of the body part of a subject, according to some embodiments;
Fig. 9a schematically illustrates a staged architecture of models utilized for conjointly analyzing the signals, according to some embodiments;
Fig. 9b schematically illustrates a parallel architecture of models utilized for conjointly analyzing the signals, according to some embodiments; and
Fig. 10 schematically illustrates a graph including IG, PG, and EG signals, according to some embodiments.

### Detailed Description of the Drawings

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

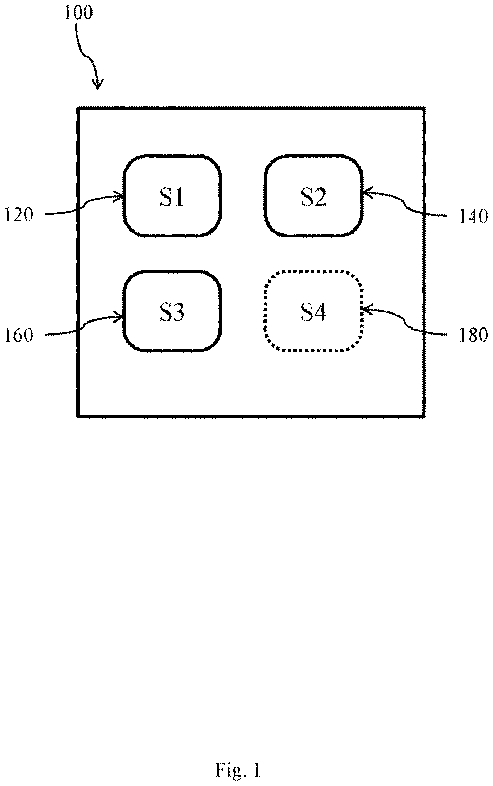

Reference is now made to Fig. 1, which schematically illustrates a device 100, according to some embodiments. The device 100 includes an EG sensor 120, an IG sensor 140, and a PG sensor 160, and, optionally, the device includes one or more additional sensors 180.

Each of the EG sensor 120, IG sensor 140, PG sensor 160, and optionally the additional sensor 180 are integrated with or connectable to an interface for acquiring signals from the body of the subjects. According to some embodiments, the EG sensor 120 and IG sensor 140 are connected to a shared set of electrodes (not shown), while the PG sensor 160 is connected to an interface, such as a membrane.

The EG sensor 120 is a surface electrical potential differentials sensor, configured to measure electrical activity of a body part and to provide/generate at least one EG signal indicative of the electrical activity of the body part.

The IG sensor 140 is an impedance sensor, configured to measure the electrical impedance of the body part and to provide an IG signal indicative of the electrical impedance of the body part.

The PG sensor 160 is a surface vibrations sensor configured to measure the acoustic activity of the body part and to provide a PG signal indicative of the acoustic activity of the body part.

The device 100 is configured to obtain the EG signal, IG signal, and PG signal for facilitating a conjoint analysis of the signal to allow for an assessment of a condition of the body part or of the subject by deriving at least one datum based on all of the EG signal, IG signal, and PG signal.

According to some embodiments, the additional sensor 180 is a predefined/preconfigured sensor, such as a temperature sensor, an oximetry sensor, or the like.

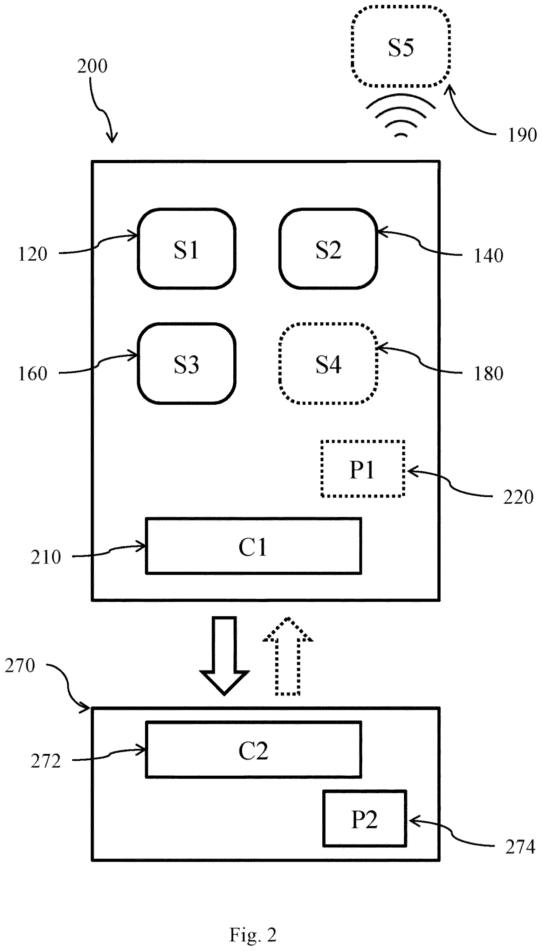

Reference is now made to Fig. 2, which schematically illustrates a device 200 in communication with an external device 270, according to some embodiments. Similarly to the device 100 of Fig. 1, the device 200 includes an EG sensor 120, an IG sensor 140, and a PG sensor 160, and, optionally, the device includes one or more additional sensor 180. Additionally, the device 200 includes a communication unit or module 210 configured to obtain the EG signal, IG signal, and PG signal and to communicate the signals to an external communication unit or module 272 of an external device 270.

The external device 270 includes the external communication unit or module, configured to receive the EG signal, IG signal, and PG signal from the communication unit or module 210 of the device 200 and to provide the signal to a processor 274. In this embodiment, the processor 274 is configured to perform the conjoint analysis.

Optionally, the external device 270 communicated or is adapted to communicate certain operative information, such as calibration information, operation commands, wake-up commands, sleep commands, or the like with the device 200, to control the operation thereof and/or to confirm an operational status, such as a connection establishment status.

Optionally, the device 200 includes an internal processor 220 configured to perform certain pre-processing procedures on one or more of the EG signal, IG signal, and PG signal.

According to some embodiments, the device 200 is connectible, by wires or wirelessly, to an external additional sensor 190.

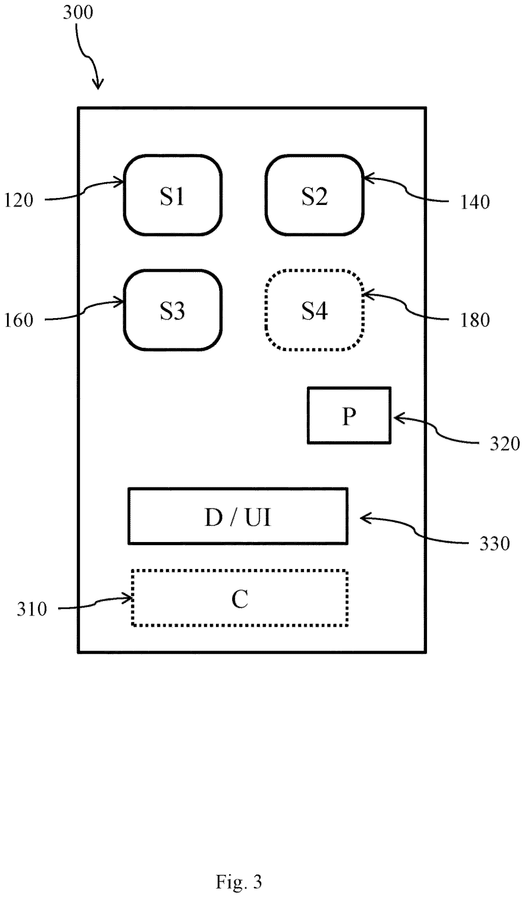

Reference is now made to Fig. 3, which schematically illustrates a device 300 with an integrated processor 320, according to some embodiments.

The device 300, similar to the device 100 of Fig. 1 and the device 200 of Fig. 2, includes an EG sensor 120, an IG sensor 140, and a PG sensor 160, and, optionally, the device includes one or more additional sensor 180. The integrated processor 320 is configured to obtain the EG signal, IG signal, and PG signal from the sensors and perform the conjoint analysis of the sensors. Furthermore, the integrated processor 320 is configured to obtain one or more additional signals from the one or more additional sensor(s) 180, if present.

The device 300 further includes a user interface 330, for obtaining inputs from a user and for providing outputs to the user. The user interface includes one or more of a display, a touch display, a light indicator, a sound indicator, a microphone, a vibration mechanism, one or more buttons, or the like.

In some embodiments, the user interface, particularly an output mechanism, such as the display, the light indicator, the sound indicator, and/or the vibration mechanism are utilized for providing biofeedback to the user based on one or more of the EG signal, IG signal, and PG signal or a result of the conjoint analysis thereof or a partial analysis thereof.

Optionally, the device 300 further includes a communication unit or module 310 configured to be communicatively connectible to communication modules of other devices, for example for receiving operation or configuration information, and/or for transmitting information related to one or more of the EG signal, IG signal, and PG signal or a result of the conjoint analysis thereof or a partial analysis thereof.

In the following embodiments, the reference to a device, such as device 201, is interchangeable with a reference to a device 200 as in Fig. 2, and further interchangeable with a reference to a device 300 corresponding to Fig. 3, or a device 100, corresponding to Fig. 2.

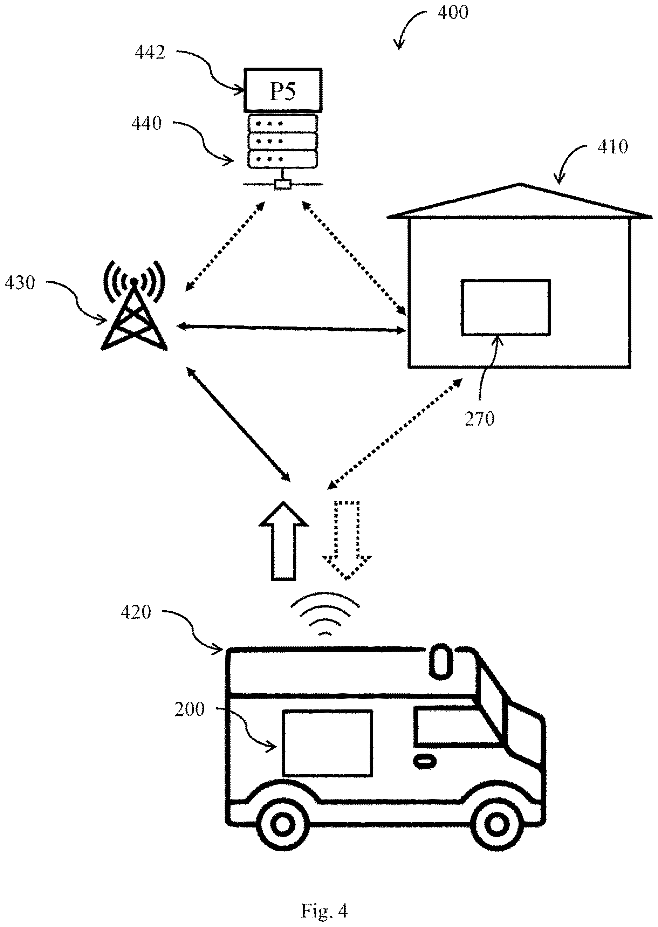

Reference is now made to Fig. 4, which schematically illustrates a system 400 including a device 200 operable in a mobile setting, according to some embodiments. In mobile settings, the device 200 and the external device 270 are movable in relation to one another and they can be distanced from one another. As illustrated, the device 200 is operable in a mobile vehicle, such as an ambulance 420, while the external device is remote from the device 200 and is operable in a clinic 410. According to some embodiments, the device 200, either directly or through a relay in the ambulance 420 communicates with the external device 270. As demonstrated in Fig. 4, the device 200 communicates wirelessly through a cellular network 430 or a private wireless network with the external device 270.

Optionally, the device 200 or a relay in the ambulance 420 communicates wirelessly directly with the external device 270 or with a relay in the clinic 410. Optionally, a server 440 is in communication with the device 200 for receiving one or more of the signals or information related to one or more of the signals, and further optionally, the server 440 includes, or is connected to a server processor 442 configured to perform the conjoint analysis or parts thereof.

According to some embodiments, the communication between the device 200 and the external device 270 includes one or more of a cellular network (directly or via a mobile device), through a wireless network, or through a wired network, via the internet, and/or via an intranet.

According to some embodiments, the device 200 is located in a home of a user while the external device 270 is located with a caregiver, in an ambulance, in a hospital, or in a clinic.

According to some embodiments, the device 200 is located in an ambulance while the external device 270 is located in a hospital. According to some embodiments, the device 200 is located in a doctor's office while the external device 270 is located with a caregiver, in a hospital, or in an ambulance.

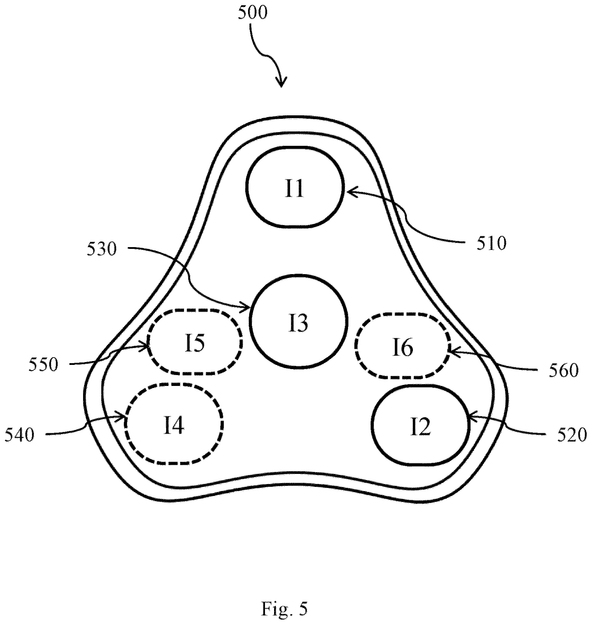

Reference is now made to Fig. 5, which schematically illustrates a patch 500 with interfaces, according to some embodiments. According to some embodiments, the patch is integrated with or removably attachable to a device, such as device 100, 200, 300, wherein the patch 500 includes interfaces configured to interface with the body of the subject and provide signals to the sensors. As illustrated in figure 5, a first electrode 510, and a second electrode 520 are electrodes, both configured to facilitate EG measurements and IG measurements. In other words, the first electrode 510, and the second electrode 520 are shared IG/EG electrodes.

The patch 500 also includes a surface vibrations interface, such as an acoustic membrane 530, configured to facilitate PG measurements.

Optionally, the patch further includes an additional electrode 540, which is either a shared IG/EG electrode, a dedicated IG electrode, or a dedicated EG electrode.

Optionally, the patch includes interfaces for providing signals to additional sensors, such as an optode 550 or a thermistor 560.

The interfaces are connected or connectible with respective sensors.

According to some embodiments, a surface of the device or a patch attachable or appliable to the subject has a length of up to 12 cm and a width of up to 12 cm. According to some embodiments, a surface of the device or a patch attachable or appliable to the subject has a length of 6 cm and a width of 6 cm. According to some embodiments, the patch has a depth/height of up to 1 cm, and preferably 0.6 cm.

According to some embodiments, one or more of the electrodes, particularly interfaces for IG and/or EG sensors, have a diameter of 24mm.

According to some embodiments, an interface of the PG sensor has a diameter of 30mm.

According to some embodiments, the device is configured to be wearable by the subject. According to some embodiments, the device is attached to the user/subject using one or more of the following attachment methods: lanyard, for example with a wired connection to sensors, a suction cup with an active vacuum or a passive suction, the device is covered completely or partially by a tape, a separate glue member between the device and the skin, a double-sided adhesive, using an attachment on the skin configured to reversibly hold the device or the patch in place, eTattoo/e-Ink, or the like.

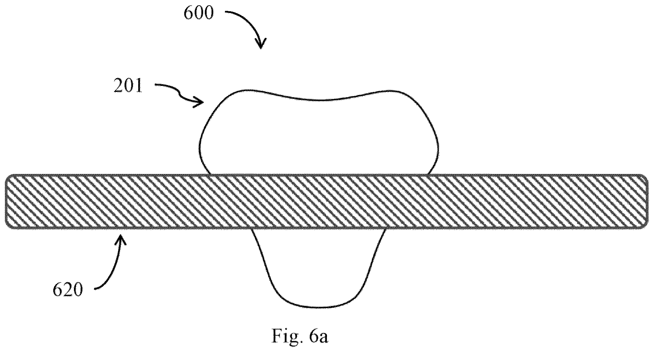

Reference is now made to Fig. 6a, which schematically illustrates a wearable system 600 including a device 201 and a strap 620, according to some embodiments. The strap 620 is configured to hold the device 201 in place and pushed against the skin of the subject to facilitate measurements of IG, PG, and EG signals.

According to some embodiments, the strap 620 is configured to be worn around the upper torso, around a limb, mid-torso, lower torso, neck, hip, or head of the subject, based on a target body part or physiological system to be monitored/measured.

According to some embodiments, the device 201 or patch is configured to be attached to the strap by pressure applied by the strap 620 and friction between the strap 620 and the device 201.

According to some embodiments, the back side of the device 201 and/or a corresponding inner part of the strap 620, includes a high-friction material, such as rubber or silicone.

According to some embodiments, the device 201 or patch is attached to the strap 620 using adhesion. According to some embodiments, the device 201 or patch are attached to the strap 620 using a reversible attachment mechanism, such as a magnetic attachment, a receptacle and a counter-part, hook and loop fastening mechanism (such as Velcro ^{®}), or the like.

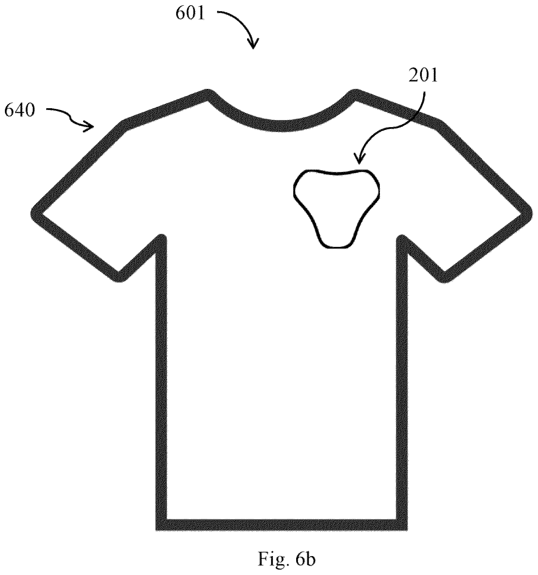

Reference is now made to Fig. 6b, which schematically illustrates a wearable system 601 including a device 201 attached to a textile item, particularly a shirt 640, according to some embodiments.

According to some embodiments, the device 201 or patch is attached to or integrated into the textile item, e.g. a shirt 640 using adhesion. According to some embodiments, the device 201 or patch are attached to the textile item, e.g. a shirt 640 using a reversible attachment mechanism, such as a magnetic attachment, a receptacle, and a counter-part, hook and loop fastening mechanism (such as Velcro ^{®}), or the like.

According to some embodiments, the textile item is a shirt, a cap, a scarf, pants, underpants, braw, gloves, socks, and/or a vest.

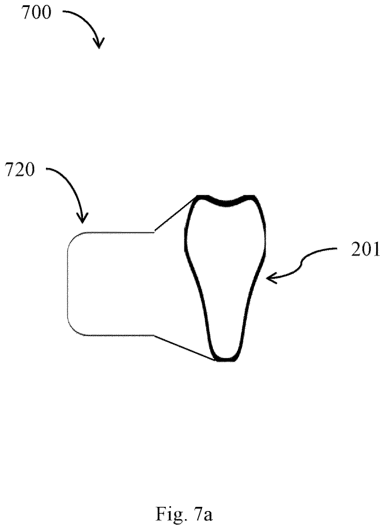

Reference is now made to Fig. 7a, which schematically illustrates a handheld apparatus 700, according to some embodiments. The device 201 or patch is attached or attachable to a handle 720 which facilitates positioning the device 201 to a specific area on the body of the subject for performing the measurements or monitoring. According to some embodiments, the user interface, including input means and/or output means are integrated into the handle 720.

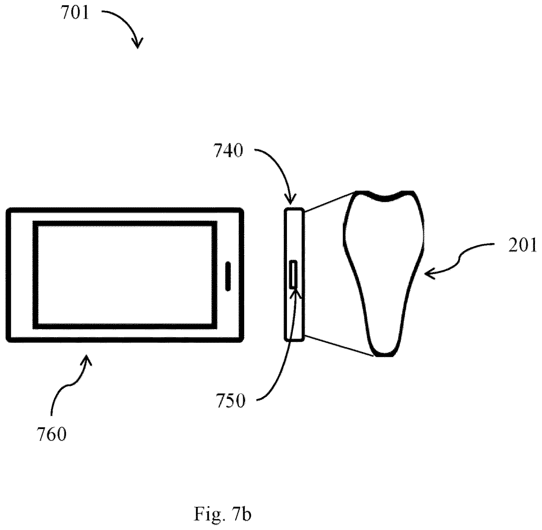

Reference is now made to Fig. 7b, which schematically illustrates a system 701 including a device 201 with a docking receptacle 740 for a mobile device 760, according to some embodiments. The receptacle 740 is configured to hold a mobile device 760, such as a smartphone, and a connector 750 is configured to facilitate communication between the device 201 and the mobile device 760. According to some embodiments, the mobile device 760 functions similarly to an external device 300 brought above. According to some embodiments, the user interface of the mobile device 760 is utilizable for viewing information regarding one or more of the signals or a result of conjoint analysis. According to some embodiments, the user interface of the mobile device 760 is configured to facilitate control over the operation or function of the device 201.

According to some embodiments, the mobile device 760 is configured to relay information between the device 201 and an external device 300 using a communication module in the mobile device 760.

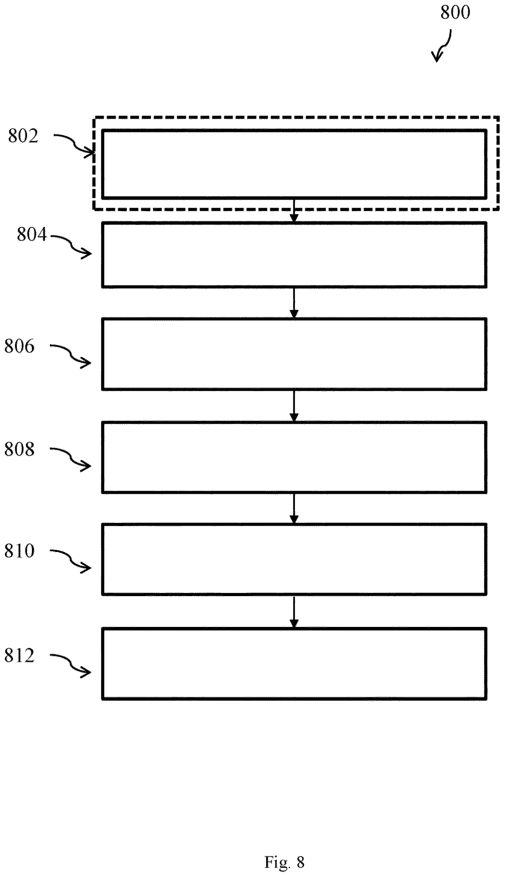

Reference is now made to Fig. 8, which schematically illustrates an algorithm, processor configuration or a method 800 for measuring and/or monitoring biological signals of the body part of a subject, according to some embodiments. Initially, optionally, demographic and/or medical information of the subject are acquired 802, the device/patch is applied at a target location on the body of the subject 804, and EG, IG, and PG signals are acquired 806 and preprocessed 808.

According to some embodiments, the pre-processing 808 of the signals involves applying one or more of filtration, noise-reduction, transformation, modulation, smoothing, derivation, and/or normalization.

Then, the preprocessed signals as well as the demographic and/or medical information are fed to a computational model for conjoint analysis 810, from which inference of results is performed 812.

According to some embodiments, the acquisition of EG, IG, and PG signals includes acquiring a baseline measurement of the EG, IG, and PG signals, then acquiring snapshots of new EG, IG, and PG signals. According to some embodiments, the acquisition of snapshots of EG, IG, and PG signals is continuous and the method 800 operates continuously in a one-way feed-forward mode. According to other embodiments, the results are fed back to an intermediate stage in the method to create a closed loop, for example, by feeding back the previous inferred information back to the model as an input thereto or by triggering the acquisition of new EG, IG, and PG signals based on the results of a previous inference.

According to some embodiments, the method 800 forms an "inference stage" in the training method of the model, which includes obtaining baseline data and time-series snapshot data of EG, IG, and PG signals, then, pre-processing of the EG, IG, and PG signals is performed and the information is used for physiological parameter estimation and feature extraction, then, a model is generated by deriving generalized representations of relations between features and outcomes based on previously annotated data, and finally, the model is used in an inference stage based on the method 800.

According to some embodiment, one model is used for the conjoint analysis, such that all the EG, IG, and PG signals as well as additional information, such as demographic and/or medical information, are fed to the model for performing the conjoint analysis.

According to other embodiments, several models are used in various structures for performing the conjoint analysis.

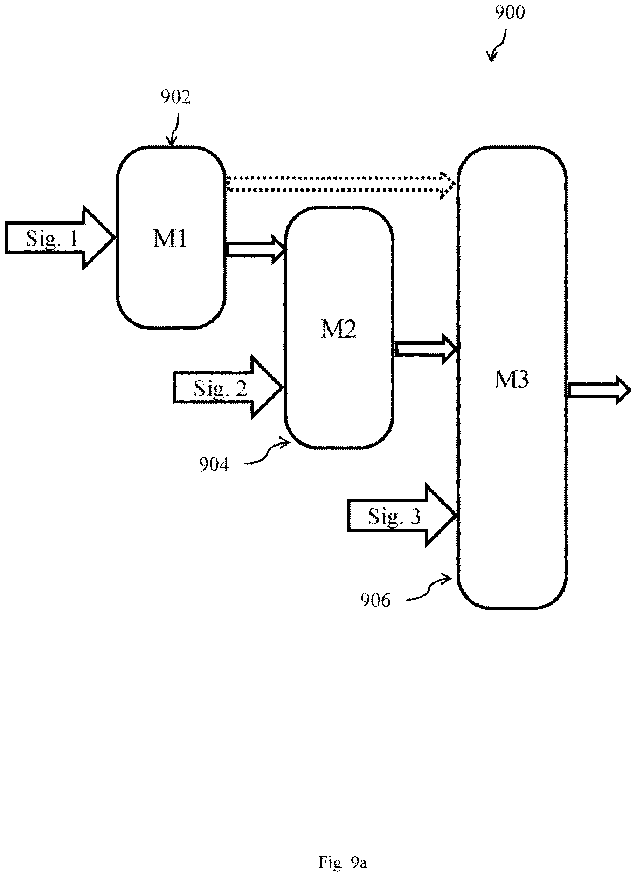

Reference is now made to Fig. 9a, which schematically illustrates a staged architecture 900 of models utilized for conjointly analyzing the signals, according to some embodiments. Initially, a first signal, such as an EG signal, is fed to a first model 902, then, the outcome of the first model 902 is fed to a second model 904 along with a second signal, such as an IG signal, then, the outcome of the second model 904, and optionally the outcome of the first model 902, are fed into a third model 906 along with a third signal, such as a PG signal for deriving a datum based on a combined conjoint analysis of all of the EG, IG, and PG signals and optionally the demographic and/or medical information.

According to some embodiment, results of intermediate stages/models are also used as data and are outputted for the user.

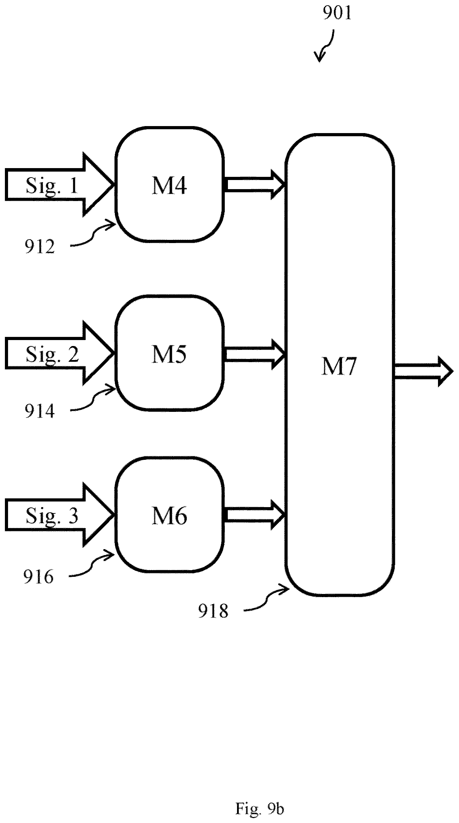

Reference is now made to Fig. 9b, which schematically illustrates a parallel architecture 901 of models utilized for conjointly analyzing the signals, according to some embodiments. The first signal , such as an EG signal, is fed to a fourth model 912, a second signal, such as an IG signal, is fed to a fifth model 914, a third signal, such as a PG signal, is fed to a sixth model 916, then the outcomes of the fourth model 912, fifth model 914, and sixth model 916 are fed into a seventh model 918 for deriving a datum based on a combined conjoint analysis of all of the EG, IG, and PG signals and optionally the demographic and/or medical information which is fed to one or more of the models.

According to some embodiments, one or more of the intermediate models brought above particularly models 902, 904, 906, 912, 914, 916, and 918 can include multiple-stacked models. According to some embodiments, one or more of the models brought above, particularly models 902, 904, 906, 912, 914, 916, and 918 can receive more than one of the IG, PG, or EG signals. According to some embodiments, the conjoint analysis is performed such that an analysis of a third signal is performed based on an outcome of an analysis of a second signal, and the analysis of the second signal is performed based on an outcome of an analysis of a first signal. The first signal, second signal, and third signal correspond to the IG, PG, and EG signals mutually exclusively at different orders according to different embodiments.

According to the invention, the conjoint analysis is performed such that an analysis of a third signal is performed based on an outcome of an analysis of a second signal and an analysis of a first signal or based on a conjoint analysis of a second signal and a first signal. The first signal, second signal, and third signal correspond to the IG, PG, and EG signals mutually exclusively at different orders according to different embodiments.

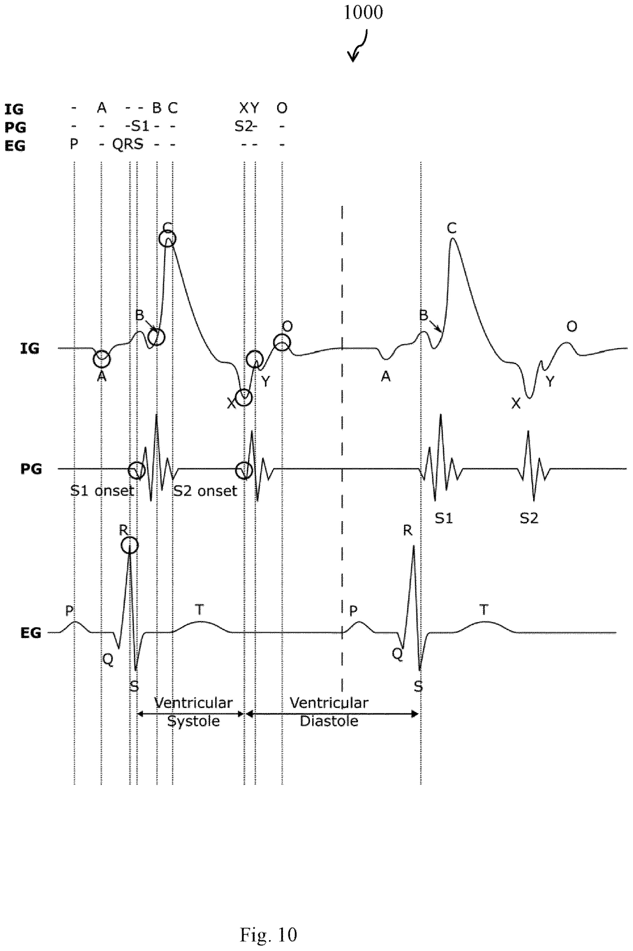

Reference is now made to Fig. 10, which schematically illustrates a graph 1000 including IG, PG, and EG signals, according to some embodiments. The EG signal is analyzed for extracting a P-point, a Q-point, an R-point, an S-point, and/or a T-point, the PG signal is analyzed, for extracting an S1 onset, an S2 onset, an S3 onset (not shown), an S4 onset (not shown), and the IG signal is analyzed for extracting an A-point, a B-point, a C-point, an X-point, a Y-point, and/or an O-point.

According to some embodiments, a segment of interest in the PG signal is the S3 sound segment, which is caused by a low-frequency brief vibration occurring in the early diastole at the end of the rapid diastolic filling period of the right or left ventricle. According to some embodiments, the S3 segment is detected by identifying the onset and the offset of a segment with high signal energy in a frequency range associated with the S3 heart sound.

According to some embodiments, a segment of interest in the PG signal is the S4 sound segment, which, if present, is caused by atrial contraction. According to some embodiments, the S4 sound segment is detected by identifying the onset and the offset of a segment with high signal energy in a frequency range associated with the S4 heart sound.

The P-point corresponds to atrial depolarization, the A-point corresponds to an atrial contraction, an R-point corresponds to ventricular depolarization, the S1 onset corresponds to a closing of the mitral valve, the B-point corresponds to an opening of the aortic valve, a C-point corresponds to a maximal ventricular contraction or maximal systole flow, an S2 onset and an X-point correspond to a closing of the aortic valve, the Y-point corresponds to a closing of the pulmonary valve, and the O-point corresponds to an opening of a mitral valve.

According to some embodiments, the conjoint analysis includes determining segments based on two detected points, wherein both points are extracted from the analysis of the same signal.

According to some embodiments, the segments include one or more of an atrial systole segment from the P-point to the S-point or the S1 onset, an isovolumetric contraction segment from the R-point to the B-point, a ventricular ejection segment from the B-Point to the S2 onset or the X-point, an isovolumetric relaxation segment from the S2 onset or the X-point to the O-point, ventricular systole from the S-point or the S1 onset to the S2 onset or the X-point, and/or a ventricular diastole segment from an S2 onset or an X-point to an S-point.

According to the invention, the conjoint analysis includes determining segments based on two detected points, wherein one of the points is extracted from the analysis of one signal while the other point is extracted from another signal.

Wherein at least part of the analysis of the EG signal, the PG signal, and/or the IG signal is based on detected points or segments in the other signals.

Unless specifically stated otherwise, as apparent from the following discussions, it is appreciated that throughout the specification discussions utilizing terms such as "processing", "computing", "calculating", "determining", "estimating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices. In addition, the term "plurality" may be used throughout the specification to describe two or more components, devices, elements, parameters, and the like.

Embodiments of the present disclosure may include apparatuses for performing the operations herein. This apparatus may be specially constructed for the desired purposes, or it may include a general-purpose computer selectively activated or reconfigured by a computer program stored in the computer. Such a computer program may be stored in a computer-readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read-only memories (EEPROMs), magnetic or optical cards, or any other type of media suitable for storing electronic instructions, and capable of being coupled to a computer system.

The disclosure may be described in the general context of computer-executable instructions, such as program modules, being executed by a computer. Generally, program modules include routines, programs, objects, components, data structures, and so forth, which perform particular tasks or implement particular abstract data types. The disclosure may also be practiced in distributed computing environments where tasks are performed by remote processing devices that are linked through a communications network. In a distributed computing environment, program modules may be located in both local and remote computer storage media including memory storage devices.

According to some embodiments, one or more of the computational models could be selected and/or adjusted based on cohort or stratified categorization of the user, for example, based on one or more of the following information: age, gender, height, weight, clinical state, clinical history, medication, comorbidities, genetic information, or other demographic information.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" or "comprising," when used in this specification, specify the presence of stated features, integers, steps, operations, elements, or components, but do not preclude or rule out the presence or addition of one or more other features, integers, steps, operations, elements, components, or groups thereof.

While a number of exemplary aspects and embodiments have been discussed above, those of skill in the art will recognize certain modifications, additions, and sub-combinations thereof. It is therefore intended that the following appended claims and claims hereafter introduced be interpreted to include all such modifications, additions, and sub-combinations as are within their scope.

### Reference Signs

- 100,200,201,300: device
- 120: EG sensor
- 140: IG sensor
- 160: PG sensor
- 180: additional sensor
- 190: external additional sensor
- 210: communication module
- 220: internal processor
- 270: external device
- 272: external communication module
- 274: processor
- 310: communication module
- 320: integrated processor
- 330: user interface
- 400: system
- 410: clinic
- 420: ambulance
- 430: cellular network
- 440: server
- 442: server processor
- 500: patch
- 510: first electrode
- 520: second electrode
- 530: acoustic membrane
- 540: additional electrode
- 550: optode
- 560: termistor
- 600, 601: wearable system
- 620: band
- 640: shirt
- 700, 701: system
- 720: handle
- 740: receptacle
- 750: connector
- 760: mobile device
- 800: method
- 802, 804, 806, 808, 810, 812: method step
- 900: staged architecture
- 901: parallel architecture
- 902: first model
- 904: second model
- 906: third model
- 912: fourth model
- 914: fifth model
- 916: sixth model
- 918: seventh model
- 1000: graph

## Claims

1. A device (100, 200, 201, 300) for measuring and/or monitoring biological signals of a body part of a subject, the device (100, 200, 201, 300) comprising:
- an electro-gram, EG sensor (120), configured to measure an electrical activity of the body part and to provide at least one EG signal indicative of the electrical activity of the body part,
- a phono-gram, PG sensor (160), configured to measure an acoustic activity of the body part and to provide a PG signal indicative of the acoustic activity of the body part,
- an impedance-gram, IG sensor (140), configured to measure an electrical impedance of the body part and to provide an IG signal indicative of the electrical impedance of the body part, and
- a processor (220) configured to:
obtain the EG signal from the EG sensor (120),
obtain the PG signal from the PG sensor (160),
obtain the IG signal from the IG sensor (140), and to
conjointly analyze the EG signal, the PG signal, and the IG signal to allow for an assessment of a predefined condition of the body part or the subject by deriving at
least one datum based on all of the EG signal, the PG signal, and the IG signal,
wherein the processor is further configured to perform the conjoint analysis by performing segment detection based on two of the EG signal, the PG signal, or the IG signal to detect a segment of interest and analyzing the other signals based on the detected segment of interest, wherein performing segment detection comprises segmenting the signal to predefined sections corresponding to phases within a predefined biological cycle, wherein analyzing the other signals based on the segmentation comprises identifying characteristic properties in one or more of the other signals based on the detected segment,
wherein performing segment detection comprises detecting a section defined by two points, wherein one of the points is detected by analyzing one of the signals while the other point is detected by analyzing a second signal, and analyzing the other signals based on the segmentation comprises analyzing a third signal based on the detected section or the two points.

2. The device (100, 200, 201, 300) of claim 1, wherein the conjoint analysis comprises deriving data from each of the signals, wherein the data derived from different signals include redundancy therebetween,
optionally, wherein the conjoint analysis further comprises estimating a signal quality or a confidence parameter of a selected one of the EG signal, the PG signal, or the IG signal by comparing a datum derived therefrom with a datum derived from one or more of the other signals as reference signals, preferably by:
determining a reference timing datum from the reference signals, determining a timing datum of the selected signal, and comparing the timing datum of the selected signal with the reference timing datum from the reference signals, or by
estimating a first datum based on a physiological parameter from the reference signals, estimating a second datum based on the same physiological parameter from the selected signal, and determining a discrepancy value between the datum estimated from the selected signal and the datum estimated from the reference signals.

3. The device (100, 200, 201, 300) of any of claims 1 or 2, wherein the conjoint analysis comprises:
determining a first physiological parameter from one or more of the EG signal, the PG signal, or the IG signal,
determining a second physiological parameter from the other one or more of the EG signal, the PG signal, or the IG signal, and
deriving a third physiological parameter based on the first physiological parameter and the second physiological parameter,
wherein the first parameter, the second parameter, and the third parameter are different from one another,
wherein the first parameter, the second parameter, and the third parameter are the same parameter, or
wherein the first parameter and the second parameter are the same parameter, and the third parameter is a different parameter.

4. The device (100, 200, 201, 300) of any of claims 1 to 3, wherein a feature derived from one of the signals is derived based on another feature derived from another signal.

5. The device (100, 200, 201, 300) of claim 4, wherein the conjoint analysis further comprises:
providing the derived plurality of features to a detection and/or prediction model, wherein the detection and/or prediction model preferably corresponds to a machine-learning model, and
generating, from the detection and/or prediction model, a score or a value indicative of a probability of the predefined condition of the body part.

6. A system for measuring and/or monitoring biological signals of the body part of a subject, the system comprising:
a wearable device (100, 200, 201, 300) comprising:
- an electro-gram, EG sensor (120), configured to measure an electrical activity of the body part and to provide at least one EG signal indicative of the electrical activity of the body part,
- a phono-gram, PG sensor (160), configured to measure an acoustic activity of the body part and to provide a PG signal indicative of the acoustic activity of the body part, and
- an impedance-gram, IG sensor (140), configured to measure an electrical impedance of the body part and to provide an IG signal indicative of the electrical impedance of the body part,
a system communication unit (272) adapted to communicate with a communication unit (210) of the wearable device (100, 200, 201, 300) to obtain the EG signal, the PG signal, and the IG signal therefrom, and
a processor (274, 442), configured to:
obtain the EG signal, the PG signal, and the IG signal from the system communication unit (272), and
conjointly analyze the EG signal, the PG signal, and the IG signal to allow for an assessment of a predefined condition of the body part by deriving at least one datum based on all of the EG signal, the PG signal, and the IG signal,
wherein the processor is further configured to perform the conjoint analysis by performing segment detection based on one or more of the EG signal, the PG signal, or the IG signal to detect a segment of interest and analyzing the other signals based on the detected segment of interest, wherein performing segment detection comprises segmenting the signal to predefined sections corresponding to phases within a predefined biological cicle, wherein analyzing the other signals based on the segmentation comprises identifying characteristic properties in one or more of the other signals based on the detected segment,
wherein performing segment detection comprises detecting a section defined by two points, wherein one of the points is detected by analyzing one of the signals while the other point is detected by analyzing a second signal, and analyzing the other signals based on the segmentation comprises analyzing a third signal based on the detected section or the two points.

7. The system of claim 6, wherein the conjoint analysis further comprises:
deriving data from each of the signals, wherein the data derived from different signals include redundancy therebetween, optionally, wherein the conjoint analysis further comprises estimating a signal quality or a confidence parameter of a selected one of the EG signal, the PG signal, or the IG signal by comparing a datum derived therefrom with a datum derived from one or more of the other signals as reference signals, preferably by:
determining a reference timing datum from the reference signals, determining a timing datum of the selected signal, and comparing the timing datum of the selected signal with the reference timing datum from the reference signals, or by estimating a first datum based on a physiological parameter from the reference signals, estimating a second datum based on the same physiological parameter from the selected signal, and determining a discrepancy value between the datum estimated from the selected signal and the datum estimated from the reference signals.

8. The system of claim 6 or 7, wherein the conjoint analysis comprises
determining a first physiological parameter from one or more of the EG signal, the PG signal, or the IG signal,
determining a second physiological parameter from the other one or more of the EG signal, the PG signal, or the IG signal, and
deriving a third physiological parameter based on the first physiological parameter and the second physiological parameter,
wherein the first parameter, the second parameter, and the third parameter are different from one another,
wherein the first parameter, the second parameter, and the third parameter are the same parameter, or
wherein the first parameter and the second parameter are the same parameter, and the third parameter is a different parameter.

## Patentansprüche

1. Vorrichtung (100, 200, 201, 300) zum Messen und/oder Überwachen biologischer Signale eines Körperteils eines Subjekts, wobei die Vorrichtung (100, 200, 201, 300) Folgendes umfasst:
- einen Elektrogramm-, EG-,Sensor (120), der so konfiguriert ist, dass er eine elektrische Aktivität des Körperteils misst und mindestens ein EG-Signal liefert, das die elektrische Aktivität des Körperteils anzeigt,
- einen Phonogramm-, PG-,Sensor (160), der so konfiguriert ist, dass er eine akustische Aktivität des Körperteils misst und ein PG-Signal liefert, das die akustische Aktivität des Körperteils anzeigt,
- einen Impedanzgramm-, IG-,Sensor (140), der so konfiguriert ist, dass er eine elektrische Impedanz des Körperteils misst und ein IG-Signal liefert, das die elektrische Impedanz des Körperteils anzeigt, und
- einen Prozessor (220), der konfiguriert ist zum:
Erhalten des EG-Signals vom EG-Sensor (120),
Erhalten des PG-Signals vom PG-Sensor (160),
Erhalten des IG-Signals vom IG-Sensor (140), und
gemeinsamen Analysieren des EG-Signals, des PG-Signals und des IG-Signals, um eine Bewertung eines vordefinierten Zustands des Körperteils oder des Subjekts zu ermöglichen, indem mindestens ein Datenwert auf der Grundlage aller des EG-Signals, des PG-Signals und des IG-Signals abgeleitet wird,
wobei der Prozessor ferner so konfiguriert ist, dass er die gemeinsame Analyse durchführt, indem er eine Segmenterkennung auf der Grundlage eines oder mehrerer des EG-Signals, des PG-Signals oder des IG-Signals durchführt, um ein Segment von Interesse zu erkennen und die anderen Signale auf der Grundlage des erkannten Segments von Interesse zu analysieren, wobei die Durchführung der Segmenterkennung das Segmentieren des Signals in vordefinierte Abschnitte umfasst, die Phasen innerhalb eines vordefinierten biologischen Zyklus entsprechen, wobei das Analysieren der anderen Signale auf der Grundlage der Segmentierung das Identifizieren charakteristischer Eigenschaften in einem oder mehreren der anderen Signale auf der Grundlage des erkannten Segments umfasst, wobei das Durchführen der Segmenterkennung das Erkennen eines durch zwei Punkte definierten Abschnitts umfasst, wobei einer der Punkte durch Analysieren eines der Signale erkannt wird, während der andere Punkt durch Analysieren eines zweiten Signals erkannt wird, und das Analysieren der anderen Signale auf der Grundlage der Segmentierung das Analysieren eines dritten Signals auf der Grundlage des erkannten Abschnitts oder der zwei Punkte umfasst.

2. Vorrichtung (100, 200, 201, 300) nach Anspruch 1, wobei die gemeinsame Analyse das Ableiten von Daten aus jedem der Signale umfasst, wobei die aus verschiedenen Signalen abgeleiteten Daten eine Redundanz dazwischen umfassen,
optional, wobei die gemeinsame Analyse ferner das Schätzen einer Signalqualität oder eines Konfidenzparameters eines ausgewählten Signals aus dem EG-Signal, dem PG-Signal oder dem IG-Signal umfasst, indem ein davon abgeleiteter Datenwert mit einem Datenwert verglichen wird, der von einem oder mehreren der anderen Signale als Referenzsignale abgeleitet wurde, vorzugsweise durch:
Bestimmen eines Referenzzeitdatenwerts aus den Referenzsignalen, Bestimmen eines Zeitdatenwerts des ausgewählten Signals und Vergleichen des Zeitdatenwerts des ausgewählten Signals mit dem Referenzzeitdatenwert aus den Referenzsignalen, oder durch
Schätzen eines ersten Datenwerts auf der Grundlage eines physiologischen Parameters aus den Referenzsignalen, Schätzen eines zweiten Datenwerts auf der Grundlage desselben physiologischen Parameters aus dem ausgewählten Signal und Bestimmen eines Diskrepanzwerts zwischen dem aus dem ausgewählten Signal geschätzten Datenwert und dem aus den Referenzsignalen geschätzten Datenwert.

3. Vorrichtung (100, 200, 201, 300) nach einem der Ansprüche 1 oder 2, wobei die gemeinsame Analyse Folgendes umfasst:
Bestimmen eines ersten physiologischen Parameters aus einem oder mehrerer des EG-Signals, des PG-Signals oder des IG-Signals,
Bestimmen eines zweiten physiologischen Parameters aus einem anderen oder mehrerer anderer des EG-Signals, des PG-Signals oder des IG-Signals, und
Ableiten eines dritten physiologischen Parameters auf der Grundlage des ersten physiologischen Parameters und des zweiten physiologischen Parameters,
wobei der erste Parameter, der zweite Parameter und der dritte Parameter voneinander verschieden sind,
wobei der erste Parameter, der zweite Parameter und der dritte Parameter derselbe Parameter sind, oder
wobei der erste Parameter und der zweite Parameter derselbe Parameter sind und der dritte Parameter ein anderer Parameter ist.

4. Vorrichtung (100, 200, 201, 300) nach einem der Ansprüche 1 bis 3, wobei ein von einem der Signale abgeleitetes Merkmal auf der Grundlage eines anderen, von einem anderen Signal abgeleiteten Merkmals abgeleitet wird.

5. Vorrichtung (100, 200, 201, 300) nach Anspruch 4, wobei die gemeinsame Analyse ferner Folgendes umfasst:
Bereitstellen der abgeleiteten Vielzahl von Merkmalen für ein Erkennungs- und/oder Vorhersagemodell, wobei das Erkennungs- und/oder Vorhersagemodell vorzugsweise einem Modell für maschinelles Lernen entspricht, und
Erzeugen eines Ergebnisses oder eines Wertes aus dem Erkennungs- und/oder Vorhersagemodell, der eine Wahrscheinlichkeit für den vordefinierten Zustand des Körperteils angibt.

6. System zum Messen und/oder Überwachen von biologischen Signalen des Körperteils eines Subjekts, wobei das System Folgendes umfasst:
eine tragbare Vorrichtung (100, 200, 201, 300), die Folgendes umfasst:
- einen Elektrogramm-, EG-,Sensor (120), der so konfiguriert ist, dass er eine elektrische Aktivität des Körperteils misst und mindestens ein EG-Signal liefert, das die elektrische Aktivität des Körperteils anzeigt,
- einen Phonogramm-, PG-,Sensor (160), der so konfiguriert ist, dass er eine akustische Aktivität des Körperteils misst und ein PG-Signal liefert, das die akustische Aktivität des Körperteils anzeigt, und
- einen Impedanzgramm-, IG-,Sensor (140), der so konfiguriert ist, dass er eine elektrische Impedanz des Körperteils misst und ein IG-Signal liefert, das die elektrische Impedanz des Körperteils anzeigt,
eine Systemkommunikationseinheit (272), die dazu ausgelegt ist, mit einer Kommunikationseinheit (210) der tragbaren Vorrichtung (100, 200, 201, 300) zu kommunizieren, um das EG-Signal, das PG-Signal und das IG-Signal davon zu erhalten, und
ein Prozessor (274, 442), der konfiguriert ist zum:
Erhalten des EG-Signals, des PG-Signals und des IG-Signals von der Systemkommunikationseinheit (272), und
gemeinsamen Analysieren des EG-Signals, des PG-Signals und des IG-Signals, um eine Bewertung eines vordefinierten Zustands des Körperteils zu ermöglichen, indem mindestens ein Datenwert auf der Grundlage aller des EG-Signals, des PG-Signals und des IG-Signals abgeleitet wird,
wobei der Prozessor ferner so konfiguriert ist, dass er die gemeinsame Analyse durchführt, indem er eine Segmenterkennung auf der Grundlage eines oder mehrerer des EG-Signals, des PG-Signals oder des IG-Signals durchführt, um ein Segment von Interesse zu erkennen und die anderen Signale auf der Grundlage des erkannten Segments von Interesse zu analysieren, wobei die Durchführung der Segmenterkennung das Segmentieren des Signals in vordefinierte Abschnitte umfasst, die Phasen innerhalb eines vordefinierten biologischen Zyklus entsprechen, wobei das Analysieren der anderen Signale auf der Grundlage der Segmentierung das Identifizieren charakteristischer Eigenschaften in einem oder mehreren der anderen Signale auf der Grundlage des erkannten Segments umfasst, wobei das Durchführen der Segmenterkennung das Erkennen eines durch die zwei Punkte definierten Abschnitts umfasst, wobei einer der Punkte durch Analysieren eines der Signale erkannt wird, während der andere Punkt durch Analysieren eines zweiten Signals erkannt wird, und das Analysieren der anderen Signale auf der Grundlage der Segmentierung das Analysieren eines dritten Signals auf der Grundlage des erkannten Abschnitts oder der zwei Punkte umfasst.

7. System nach Anspruch 6, wobei die gemeinsame Analyse ferner Folgendes umfasst:
Ableiten von Daten aus jedem der Signale, wobei die aus verschiedenen Signalen abgeleiteten Daten eine Redundanz dazwischen umfassen, optionalerweise, wobei die gemeinsame Analyse ferner das Schätzen einer Signalqualität oder eines Konfidenzparameters eines ausgewählten Signals aus dem EG-Signal, dem PG-Signal oder dem IG-Signal umfasst, indem ein davon abgeleiteter Datenwert mit einem Datenwert verglichen wird, der von einem oder mehreren der anderen Signale als Referenzsignale abgeleitet wurde, vorzugsweise durch:
Bestimmen eines Referenzzeitdatenwerts aus den Referenzsignalen, Bestimmen eines Zeitdatenwerts des ausgewählten Signals und Vergleichen des Zeitdatenwerts des ausgewählten Signals mit dem Referenzzeitdatenwert aus den Referenzsignalen, oder durch
Schätzen eines ersten Datenwerts auf der Grundlage eines physiologischen Parameters aus den Referenzsignalen, Schätzen eines zweiten Datenwerts auf der Grundlage desselben physiologischen Parameters aus dem ausgewählten Signal und Bestimmen eines Diskrepanzwerts zwischen dem aus dem ausgewählten Signal geschätzten Datenwert und dem aus den Referenzsignalen geschätzten Datenwert.

8. System nach Anspruch 6 oder 7, wobei die gemeinsame Analyse Folgendes umfasst
Bestimmen eines ersten physiologischen Parameters aus einem oder mehrerer des EG-Signals, des PG-Signals oder des IG-Signals,
Bestimmen eines zweiten physiologischen Parameters aus einem anderen oder mehrerer anderer des EG-Signals, des PG-Signals oder des IG-Signals, und Ableiten eines dritten physiologischen Parameters auf der Grundlage des ersten physiologischen Parameters und des zweiten physiologischen Parameters,
wobei der erste Parameter, der zweite Parameter und der dritte Parameter voneinander verschieden sind,
wobei der erste Parameter, der zweite Parameter und der dritte Parameter derselbe Parameter sind, oder
wobei der erste Parameter und der zweite Parameter derselbe Parameter sind und der dritte Parameter ein anderer Parameter ist.

## Revendications

1. Dispositif (100, 200, 201, 300) pour mesurer et/ou surveiller les signaux biologiques d'une partie du corps d'un sujet, le dispositif (100, 200, 201, 300) comprenant :
- un capteur d'électrogramme, EG, (120), configuré pour mesurer une activité électrique de la partie du corps et pour fournir au moins un signal EG indiquant l'activité électrique de la partie du corps,
- un capteur de phonogramme, PG, (160), configuré pour mesurer une activité acoustique de la partie du corps et pour fournir un signal PG indiquant l'activité acoustique de la partie du corps,
- un capteur d'impédance-gramme, IG, (140), configuré pour mesurer une impédance électrique de la partie du corps et pour fournir un signal IG indiquant l'impédance électrique de la partie du corps, et
- un processeur (220) configuré pour :
obtenir le signal EG du capteur EG (120),
obtenir le signal PG du capteur PG (160),
obtenir le signal IG du capteur IG (140), et
analyser conjointement le signal EG, le signal PG et le signal IG pour permettre une évaluation d'un état prédéfini de la partie du corps ou du sujet en dérivant au moins
une donnée en se basant sur l'ensemble du signal EG, du signal PG et du signal IG, dans lequel le processeur est en outre configuré pour effectuer l'analyse conjointe en réalisant une détection de segment sur la base d'un ou de plusieurs des signaux EG, des signaux PG ou des signaux IG pour détecter un segment d'intérêt et en analysant les autres signaux sur la base du segment d'intérêt détecté, la réalisation de la détection de segment consistant à segmenter le signal en sections prédéfinies correspondant à des phases d'un cycle biologique prédéfini, l'analyse des autres signaux sur la base de la segmentation comprend l'identification de propriétés caractéristiques dans un ou plusieurs des autres signaux sur la base du segment détecté, la réalisation de la détection d'un segment comprenant la détection d'une section définie par deux points, l'un des points étant détecté par l'analyse d'un des signaux tandis que l'autre point étant détecté par l'analyse d'un deuxième signal, et l'analyse des autres signaux sur la base de la segmentation comprend l'analyse d'un troisième signal sur la base de la section détectée ou des deux points.

2. Dispositif (100, 200, 201, 300) de la revendication 1, dans lequel l'analyse conjointe comprend l'obtention des données à partir de chacun des signaux, les données obtenues à partir des différents signaux étant redondantes entre elles,
éventuellement, dans lequel l'analyse conjointe comprend en outre l'estimation d'une qualité de signal ou d'un paramètre de confiance d'un signal sélectionné parmi le signal EG, le signal PG ou le signal IG en comparant une donnée dérivée de celui-ci à une donnée dérivée d'un ou de plusieurs des autres signaux en tant que signaux de référence, de préférence :
en déterminant une donnée temporelle de référence à partir des signaux de référence, en déterminant une donnée temporelle du signal sélectionné et en comparant la donnée temporelle du signal sélectionné à la donnée temporelle de référence des signaux de référence, ou
en estimant une première donnée sur la base d'un paramètre physiologique à partir des signaux de référence, en estimant une deuxième donnée sur la base du même paramètre physiologique à partir du signal sélectionné, et en déterminant une valeur de divergence entre la donnée estimée à partir du signal sélectionné et la donnée estimée à partir des signaux de référence.

3. Dispositif (100, 200, 201, 300) de l'une des revendications 1 ou 2, dans lequel l'analyse conjointe comprend :
déterminer un premier paramètre physiologique à partir d'un ou de plusieurs des signaux EG, des signaux PG ou des signaux IG,
déterminer un deuxième paramètre physiologique à partir de l'autre un ou de plusieurs des signaux EG, des signaux PG ou des signaux IG, et
obtenir un troisième paramètre physiologique sur la base du premier paramètre physiologique et du deuxième paramètre physiologique,
dans lequel le premier paramètre, le deuxième paramètre et le troisième paramètre sont différents l'un de l'autre,
dans lequel le premier paramètre, le deuxième paramètre et le troisième paramètre sont les mêmes paramètres, ou
dans lequel le premier paramètre et le deuxième paramètre sont identiques et le troisième paramètre est différent.

4. Dispositif (100, 200, 201, 300) de l'une des revendications 1 à 3, dans lequel une caractéristique dérivée de l'un des signaux est dérivée sur la base d'une autre caractéristique dérivée d'un autre signal.

5. Dispositif (100, 200, 201, 300) de la revendication 4, dans lequel l'analyse conjointe comprend en outre :
fournir la pluralité de caractéristiques dérivées à un modèle de détection et/ou de prédiction, le modèle de détection et/ou de prédiction correspondant de préférence à un modèle d'apprentissage automatique, et
générer, à partir du modèle de détection et/ou de prédiction, un score ou une valeur indiquant une probabilité de l'état prédéfini de la partie du corps.

6. Système de mesure et/ou de surveillance des signaux biologiques de la partie du corps d'un sujet, le système comprenant :
Dispositif portable (100, 200, 201, 300) comprenant :
- un capteur d'électrogramme, EG, (120), configuré pour mesurer une activité électrique de la partie du corps et pour fournir au moins un signal EG indiquant l'activité électrique de la partie du corps,
- un capteur de phonogramme, PG, (160), configuré pour mesurer une activité acoustique de la partie du corps et pour fournir un signal PG indiquant l'activité acoustique de la partie du corps, et
- un capteur d'impédance-gramme, IG, (140), configuré pour mesurer une impédance électrique de la partie du corps et pour fournir un signal IG indiquant l'impédance électrique de la partie du corps,
une unité de communication de système (272) adaptée pour communiquer avec une unité de communication (210) du dispositif portable (100, 200, 201, 300) afin d'obtenir le signal EG, le signal PG et le signal IG à partir de ceux-ci, et
un processeur (274, 442) configuré pour :
obtenir le signal EG, le signal PG et le signal IG de l'unité de communication de système (272), et
analyser conjointement le signal EG, le signal PG et le signal IG pour permettre une évaluation d'un état prédéfini de la partie du corps en dérivant au moins une donnée en se basant sur l'ensemble du signal EG, du signal PG et du signal IG,
dans lequel le processeur est en outre configuré pour effectuer l'analyse conjointe en réalisant une détection de segment sur la base d'un ou de plusieurs des signaux EG, des signaux PG ou des signaux IG pour détecter un segment d'intérêt et en analysant les autres signaux sur la base du segment d'intérêt détecté, la réalisation de la détection de segment consistant à segmenter le signal en sections prédéfinies correspondant à des phases d'un cycle biologique prédéfini, l'analyse des autres signaux sur la base de la segmentation comprend l'identification de propriétés caractéristiques dans un ou plusieurs des autres signaux sur la base du segment détecté, la réalisation de la détection d'un segment comprenant la détection d'une section définie par les deux points, l'un des points étant détecté par l'analyse d'un des signaux tandis que l'autre point étant détecté par l'analyse d'un deuxième signal, et l'analyse des autres signaux sur la base de la segmentation comprend l'analyse d'un troisième signal sur la base de la section détectée ou des deux points.

7. Système de la revendication 6, dans lequel l'analyse conjointe comprend en outre :
l'obtention des données à partir de chacun des signaux, les données obtenues à partir des différents signaux étant redondantes entre elles, éventuellement, l'analyse conjointe comprenant en outre l'estimation d'une qualité de signal ou d'un paramètre de confiance d'un signal sélectionné parmi le signal EG, le signal PG ou le signal IG en comparant une donnée dérivée de celui-ci à une donnée dérivée d'un ou de plusieurs des autres signaux en tant que signaux de référence, de préférence :
en déterminant une donnée temporelle de référence à partir des signaux de référence, en déterminant une donnée temporelle du signal sélectionné et en comparant la donnée temporelle du signal sélectionné à la donnée temporelle de référence des signaux de référence, ou
en estimant une première donnée sur la base d'un paramètre physiologique à partir des signaux de référence, en estimant une deuxième donnée sur la base du même paramètre physiologique à partir du signal sélectionné, et en déterminant une valeur de divergence entre la donnée estimée à partir du signal sélectionné et la donnée estimée à partir des signaux de référence.

8. Système de la revendication 6 ou 7, dans lequel l'analyse conjointe comprend
déterminer un premier paramètre physiologique à partir d'un ou de plusieurs des signaux EG, des signaux PG ou des signaux IG,
déterminer un deuxième paramètre physiologique à partir de l'autre un ou de plusieurs des signaux EG, des signaux PG ou des signaux IG, et
obtenir un troisième paramètre physiologique sur la base du premier paramètre physiologique et du deuxième paramètre physiologique,
dans lequel le premier paramètre, le deuxième paramètre et le troisième paramètre sont différents l'un de l'autre,
dans lequel le premier paramètre, le deuxième paramètre et le troisième paramètre sont les mêmes paramètres, ou
dans lequel le premier paramètre et le deuxième paramètre sont identiques et le troisième paramètre est différent.
